# EUROPEAN PATENT APPLICATION

(11) **EP 2 258 166 A1**
(43) Date of publication of application: **08.12.2010**
(21) Application number: 09712642.9
(22) Date of filing: 23.02.2009
(51) Int. Cl.: A01K 67/027, A61K 31/7088, A61K 35/48, A61K 48/00, A61P 43/00, C12N 15/09, C12N 5/10

(54) **METHOD FOR PRODUCING FOUNDER ANIMAL FOR REPRODUCING ANIMALS HAVING LETHAL PHENOTYPE CAUSED BY GENE MODIFICATION**

(30) Priority: 22.02.2008 JP 2008042243; 22.08.2008 JP 2008214711
(71) Applicant: The University of Tokyo, Bunkyo-Ku Tokyo 113-8654 (JP)
(72) Inventor: NAKAUCHI, Hiromitsu, Tokyo 113-8654 (JP); KOBAYASHI, Toshihiro, Tokyo 113-8654 (JP)
(74) Representative: Docherty, Robert Charles
(86) International application number: PCT/JP2009/053232
(87) International publication number: WO 2009/104794

(57) **Abstract**

An object of the present invention is to provide a method which constantly enables organ regeneration for the purpose of achieving organ regeneration with higher efficiency. It has been discovered that, in a blastocyst complementation method, a next generation is born when a deficiencyin an organ, such as pancreas and kidney, is complemented by injection of ES cells into a generated blastocyst, and further discovered that a transgenic animal having a pancreas or a kidney thus complemented can transmit the phenotype to the next generation as a founder. This discovery has revealed that organ regeneration can be accomplished by using such a founder. Thus, the present invention achieved the above-described object.

SEQUENCE LISTING

<110> The University of Tokyo
NAKAUCHI, Hiromitsu
KOBAYASHI, Toshihiro

<120> Method for producing founder animals for breeding of animals
transformed with fatal phenotype

<130> UTO002PCT

<150> JP 2008- 42243
<151> 2008-02-22

<150> JP 2008-214711
<151> 2008-08-22

<160> 15

<170> PatentIn version 3.3

<210> 1
<211> 21
<212> DNA
<213> artificial

<220>
<223> Example 1-2: primer sequence

<400> 1
ttcatgcgac ggttttggaa c 21

<210> 2
<211> 21
<212> DNA
<213> artificial

<220>
<223> Example 1-2: primer sequence

<400> 2
ttcaacatca ctgccagctc c 21

<210> 3
<211> 18
<212> DNA
<213> artificial

<220>
<223> Example 1-2: primer sequence

<400> 3
tgtgagcgag taacaacc 18

<210> 4

<211>  20
<212>  DNA
<213>  artificial

<220>
<223>  Example 1-2: primer sequence

<400>  4
tgactttctg tgctcagagg                                                    20


<210>  5
<211>  20
<212>  DNA
<213>  artificial

<220>
<223>  Example 1-2: primer sequence

<400>  5
caatgatggc tccagggtaa                                                    20


<210>  6
<211>  24
<212>  DNA
<213>  artificial

<220>
<223>  Example 2: primer sequence

<400>  6
ccctggggat gctgtgagcc aagg                                               24


<210>  7
<211>  24
<212>  DNA
<213>  artificial

<220>
<223>  Example 2: primer sequence

<400>  7
   ccctttttct ggagactaaa taaa                                                24


<210>  8
<211>  24
<212>  DNA
<213>  artificial

<220>
<223>  Example 2: primer sequence

<400>  8
gcgaactcac acaggcgaga aacc                                               24

<210> 9
<211> 24
<212> DNA
<213> artificial

<220>
<223> Example 2: primer sequence

<400> 9
ttatcgtcga ccactgtgct gctg 24


<210> 10
<211> 24
<212> DNA
<213> artificial

<220>
<223> Example 2: primer sequence

<400> 10
ggttacctct tcctcccact ccag 24


<210> 11
<211> 24
<212> DNA
<213> artificial

<220>
<223> Example 2: primer sequence

<400> 11
cagaggagga acgagctgaa gcgc 24


<210> 12
<211> 20
<212> DNA
<213> artificial

<220>
<223> Example 5: primer sequence

<400> 12
aagggactgg ctgctattgg 20


<210> 13
<211> 21
<212> DNA
<213> artificial

<220>
<223> Example 5: primer sequence

<400> 13

gtacacgttt ctcctcagga c                                                    21

<210>  14
<211>  20
<212>  DNA
<213>  artificial

<220>
<223>  Example 5: primer sequence

<400>  14
atatcacggg atgccaacgc                                                      20

<210>  15
<211>  21
<212>  DNA
<213>  artificial

<220>
<223>  Example 5: primer sequence

<400>  15
tctccagtgt gagttctctc g                                                    21

SEQUENCE LISTING

<110>  The University of Tokyo
       NAKAUCHI, Hiromitsu
       KOBAYASHI, Toshihiro

<120>  Method for producing founder animals for breeding of animals
       transformed with fatal phenotype

<130>  UTO002PCT

<150>  JP 2008- 42243
<151>  2008-02-22

<150>  JP 2008-214711
<151>  2008-08-22

<160>  15

<170>  PatentIn version 3.3

<210>  1
<211>  21
<212>  DNA
<213>  artificial

<220>
<223>  Example 1-2: primer sequence

<400>  1
ttcatgcgac ggttttggaa c                                                21


<210>  2
<211>  21
<212>  DNA
<213>  artificial

<220>
<223>  Example 1-2: primer sequence

<400>  2
ttcaacatca ctgccagctc c                                                21


<210>  3
<211>  18
<212>  DNA
<213>  artificial

<220>
<223>  Example 1-2: primer sequence

<400>  3
tgtgagcgag taacaacc                                                    18


<210>  4
<211>  20
<212>  DNA
<213>  artificial

<220>
<223>  Example 1-2: primer sequence

<400>  4
tgactttctg tgctcagagg                                                   20


<210>  5
<211>  20
<212>  DNA
<213>  artificial

<220>
<223>  Example 1-2: primer sequence

<400>  5
caatgatggc tccagggtaa                                                   20


<210>  6
<211>  24
<212>  DNA
<213>  artificial

<220>
<223>  Example 2: primer sequence

<400>  6
ccctggggat gctgtgagcc aagg                                              24


<210>  7
<211>  24
<212>  DNA
<213>  artificial

<220>
<223>  Example 2: primer sequence

<400>  7
   ccctttttct ggagactaaa taaa                                               24


<210>  8
<211>  24
<212>  DNA
<213>  artificial

<220>
<223>  Example 2: primer sequence

<400>  8
gcgaactcac acaggcgaga aacc                                              24


<210>  9
<211>  24
<212>  DNA
<213>  artificial

<220>
<223>  Example 2: primer sequence

<400>  9
ttatcgtcga ccactgtgct gctg                                              24


<210>  10
<211>  24
<212>  DNA
<213>  artificial

<220>
<223>  Example 2: primer sequence

<400>  10
ggttacctct tcctcccact ccag                                              24


<210>  11
<211>  24
<212>  DNA
<213>  artificial

<220>
<223>  Example 2: primer sequence

<400>  11
cagaggagga acgagctgaa gcgc                                              24


<210>  12
<211>  20
<212>  DNA
<213>  artificial

<220>
<223>  Example 5: primer sequence

<400>  12
aagggactgg ctgctattgg                                                   20


<210>  13
<211>  21
<212>  DNA
<213>  artificial

<220>
<223>  Example 5: primer sequence

<400>  13
gtacacgttt ctcctcagga c                                                 21


<210>  14
<211>  20
<212>  DNA
<213>  artificial

<220>

<223>  Example 5: primer sequence

<400>  14
atatcacggg atgccaacgc                                                    20


<210>  15
<211>  21
<212>  DNA
<213>  artificial

<220>
<223>  Example 5: primer sequence

<400>  15
tctccagtgt gagttctctc g                                                  21

## Description

### TECHNICAL FIELD

The present invention relates to an animal for reproduction, the animal being used for producing a desired cell-derived organ in vivo, and to a method for producing the animal.

### BACKGROUND OF THE INVENTION

In discussing regenerative medicine that is practiced in the form of cell transplantation and organ transplantation, pluripotent stem cells are highly expected. ES cells established from the inner cell mass of blastocyst stage fertilized eggs are pluripotent, and therefore used in various studies on cell differentiation. Development of differentiation control methods of inducing differentiation of such ES cells into specific cell lineages in vitro is a topic in the field of regeneration medicine research.

In the research on in vitro differentiation using embryonic stem (ES) cells, differentiation into mesoderms and ectoderms, such as hemocytes, bloodvessels, myocardia, and neurons, which differentiate during early embryogenesis, is likely to occur. However, it is known that there is a general tendency that differentiation into organs directed to the formation of complicated histogenesis through intracellular interactions during and after the middle embryogenesis is difficult.

For example, a metanephros, which is an adult kidney of mammals, develops from intermediate mesoderm during middle embryogenesis. Specifically, the development of kidney is initiated by the interaction between two components, which are a metanephric mesenchymal cell and a ureteric bud epithelium, and an adult kidney is completed at the end through differentiations into a number of types of functional cells, which is as large as dozens and cannot be seen in other organs, and through the formation of a complicated nephron structure, which is mainly based on a glomerulus and a renal tubule, as a result of the differentiations. It is easily inferred from the timing of kidney development and the complication of the process thereof that induction of a kidney from ES cells in vitro is an extremely complex and difficult work, and the induction is considered to be actually impossible. Further, identification of somatic stem cells in organs, such as kidney, has not been established yet, and it has started to be revealed that contribution of bone marrow cells to the repair processes of injured kidney, which was once used to be actively studied, is not very significant.

When a pluripotent ES cell is injected into the inner space of a blastocyst stage fertilized egg, a resulting individual forms a chimeric mouse. There has been previously reported a rescue experiment of T-cell and B-cell lineages by blastocyst complementation, to which this technique is applied, the rescue experiment being carried out on Rag-2 knockout mouse deficient in T-cell and B-cell lineages (Non-Patent Document 1). This chimeric mouse assay is used as an in vivo assay system for verifying the differentiation of the T-cell lineage, for which no in vitro assay system is available.

The inventors have filed an application PCT/JP2008/51129 for a method of organ regeneration in association with the above-described technique (Patent Document 1: WO/2008/102602). However, the method used in the above application is only effective for one generation. Accordingly, for each organ to be regenerated, complicated operations need to be repeated.

Therefore, in order to achieve organ regeneration with higher efficiency, it is necessary to develop a technique which constantly enables organ regeneration.
Patent Document 1: International Publication No. 2008/102602
Non-Patent Document 1: Chen J., et al., Proc. Natl. Acad. Sci. USA, Vol. 90, pp. 4528-4532, 1993

### DISCLOSURE OF THE INVENTION

### PROBLEM TO BE SOLVED BY THE INVENTION

A problem of the present invention is to provide a technique for organ regeneration, the technique being suitable for industrial application.

### MEANS FOR SOLVING THE PROBLEM

It has been discovered that, in a blastocyst complementation method, a next generation is born when a deficiency of an organ, such as pancreas and kidney, or a body part is complemented by injection of ES cells into a developed blastocyst, and further discovered that a transgenic animal having an organ, such as pancreas and kidney, or a body part thus complemented can transmit its phenotype to the next generation as a founder. This discovery has revealed that organ regeneration can be accomplished by using such a founder. Thus, the present invention has solved the above-described problem.

In the present invention, it has been discovered that founders can be used for efficiently obtaining a litter, the founder being obtained by transplanting ES cells or iPS cells of mouse or the like as pluripotent stem cells into knockout mice or transgenic animals (for example, mice), which are characterized by having a deficiency of organ, such as pancreas and kidney, or of a body part, so as to complement the organ, such as pancreas and kidney, or the body part.

In the present invention, it was found from the result of genotyping that knockout mice each with a pancreas complemented grow to be normal adults. The complemented knockout (also referred to as KO) mouse was expected to be theoretically a KO or hetero individual at a probability of 1/2 according to Mendelian inheritance, as being derived from breeding between the KO, which is capable of transmitting its phenotype to the next generation as a founder, and a hetero mouse. This was found to be as expected in reality.

From this, it is possible to obtain a KO individual at a probability of 100% from breeding between KO individuals in which, for example, an organ, such as pancreas and kidney, or a body part has been complemented. Therefore, it is expected that analysis using KO individuals will be able to be carried out significantly more easily.

A conventional method for producing a transgenic (Tg) animal is to introduce a transgene, which induces a deficiency of an organ, such as organ and kidney, or a body part, into an egg cell, and then to transplant a resulting egg cell. In this method, an animal having a phenotype of organ deficiency dies after birth if the organ is essential for survival. In this respect, it was discovered that a next generation is born when a deficiency of pancreas is complemented by injection of pluripotent stem cells, such as ES cells and iPS cells, into a developed blastocyst. In addition, it was also discovered that the transgenic animal with a pancreas thus complemented is capable of transmitting its phenotype to the next generation as a founder. Thus, it has been revealed that organ regeneration can be carried out using such a founder.

It should be understood that, once the method of the present invention is found to be applicable to a certain organ, appropriate modifications on the basis of previous successful cases can be applied to the organ. The reason for this is as follows. If an appropriate defective animal is available, a similar method of analysis can be applied thereto using fluorescent or the like-labeled pluripotent stem cells, such as ES cells and iPS cells, or using a labeled defective animal and pluripotent stem cells which are not labeled or with another label, as indicated in the present description, so as to reveal whether a thus constructed organ is derived from the host or from the ES cells, iPS cells, or the like. This allows a judgment whether organ construction has been successful or not. Thus, it should be understood in accordance with the same theory that a next generation animal can be reproduced.

Therefore, the present invention provides the followings.

In one aspect, the present invention provides an animal, which includes a deficiency responsible gene coding for a factor which causes a deficiency of any one of an organ and a body part and gives any one of no possibility of survival and difficulty in survival if the factor functions, and in which the any one of an organ and a body part is complemented by blastocyst complementation.

In one embodiment, the any one of an organ and a body part to be complemented is labeled.

In one embodiment, a method for producing the animal of the present invention comprises the steps of:
A) providing a first pluripotent stem cell having the deficiency responsible gene;
B) growing the first pluripotent stem cell into a blastocyst;
C) introducing a second pluripotent stem cell into the blastocyst so as to produce a chimeric blastocyst, the second pluripotent stem cell having an ability to complement a deficiency caused by the deficiency responsible gene; and
D) producing an individual from the chimeric blastocyst, and then selecting an individual in which the any one of an organ and a body part has been complemented by the second pluripotent stem cell.

In one embodiment, the first pluripotent stem cell is any one of an inner cell mass (ICM), a fertilized egg, and an embryo.

In one embodiment, the gene is a foreign gene, and the step A) includes introducing the foreign gene into the first pluripotent stem cell.

In one embodiment, the second pluripotent stem cell is any one of an egg cell, an ES cell, and an iPS cell.

In one embodiment, the step D) includes returning the chimeric blastocyst into a surrogate parent, causing pseudo-pregnancy thereof, and thereby producing the individual.

In one embodiment, the selecting is achieved by distinguishing an identifier derived from the second pluripotent stem cell.

In one embodiment, the second pluripotent stem cell is labeled, and the selecting includes identifying the label.

In another aspect, the present invention provides a method for producing any one of a target organ and a target body part, the method comprising the steps of:
A) providing the animal according to claim 1, in which the deficiency responsible gene codes for a factor which causes a deficiency of the any one of a target organ and a target body part;
B) obtaining an ovum from the animal, and then growing the ovum into a blastocyst;
C) introducing a target pluripotent stem cell into the blastocyst so as to produce a chimeric blastocyst, the target pluripotent stem cell having a desired genome capable of complementing a deficiency caused by the deficiency responsible gene; and
D) producing an individual from the chimeric blastocyst, and then obtaining the any one of a target organ and a body part from the individual.

In one embodiment, the step D) includes developing the chimeric blastocyst in a womb of the non-human mammal to obtain a litter, and obtaining the target organ from an individual of the litter.

In one embodiment, the target pluripotent stem cell is any one of an ES cell and an iPS cell.

In one embodiment, the target pluripotent stem cell is derived from a mouse.

In one embodiment, the any one of a target organ and a target body part is any one of a pancreas and a kidney.

In one embodiment, the animal is a mouse.

In one embodiment, the mouse is any one of a Pdx-1 knockout mouse, a Pdx1-Hes1 transgenic mouse, a Sall1 knockout mouse, and the like.

In one embodiment, the any one of an organ and a body part is completely derived from the target pluripotent stem cell.

In another aspect, the present invention provides use of an animal, which includes a gene coding for a factor which causes a deficiency of any one of an organ and a body part and gives any one of no possibility of survival and difficulty in survival if the factor functions, and in which the any one of an organ and a body part is complemented by complementation, for production of the any one of an organ and a body part.

In another aspect, provided is a set for producing any one of a target organ and a target body part, the set comprising: A) an animal, which includes a gene coding for a factor which causes a deficiency of any one of an organ and a body part and gives any one of no possibility of survival and difficulty in survival if the factor functions, and in which the any one of an organ and a body part is complemented by complementation; and B) a cell of the same type as the any one of a target organ and a body part.

In further another aspect, provided is use of any one of: a combination of a pluripotent stem cell and nucleic acids coding for a gene coding for a factor which causes a deficiency of any one of an organ and a body part and gives any one of no possibility of survival and difficulty in survival if the factor functions; and a combination of a pluripotent stem cell which has a gene coding for a factor which causes a deficiency of any one of an organ and a body part and gives any one of no possibility of survival and difficulty in survival if the factor functions and a cell which does not have the gene, the use being for production of the animal of the present invention.

### EFFECTS OF THE INVENTION

According to the present invention, a technique for organ regeneration has been provided, the technique being suitable for industrial application.

For example, the establishment of a technique for producing a founder animal allows efficient production of genetically modified (knockout or transgenic) animals which have been difficult to be maintained in conventional methods. Further, by using thus produced founder animals, manipulation using a genetically modified embryo or neonate (research focusing on organ regeneration and the like) can be carried out much more efficiently. In the meantime, although production of a transgenic animal is labor intensive, there has been a drawback that, if a lethal phenotype appears in the first generation obtained from gene transfer, analysis is allowed to be performed only once on an animal with the same position of gene insertion and copy number. However, application of this method enables survival of the first generation which is originally destine to die, thereby allowing the completely same phenotype thereof to be transmitted to the next generation. Therefore, analysis for revealing a mechanism of lethality and the like can be carried out in a completely clonal colony. Thus, it became possible to conduct experiments for the purpose of analysis using thus produced founder mice. It can be said that this is a technique which was absolutely impossible in the prior art.

### BRIEF DESCRIPTION OF THE DRAWINGS

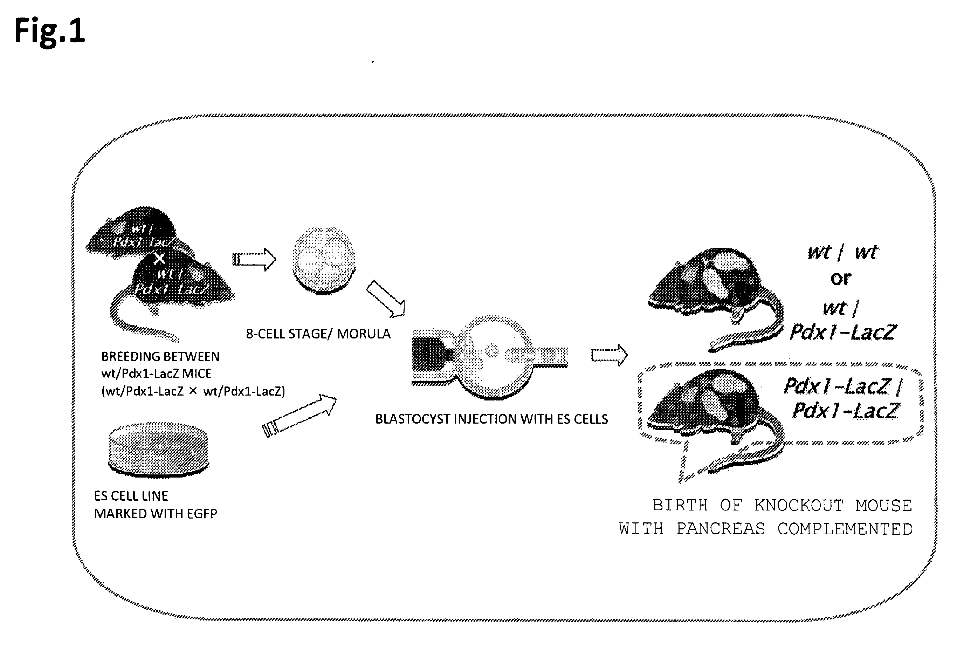

[Fig. 1] Fig. 1 shows a scheme of birth of a knockout (KO) mouse with a pancreas complemented by blastocyst complementation.

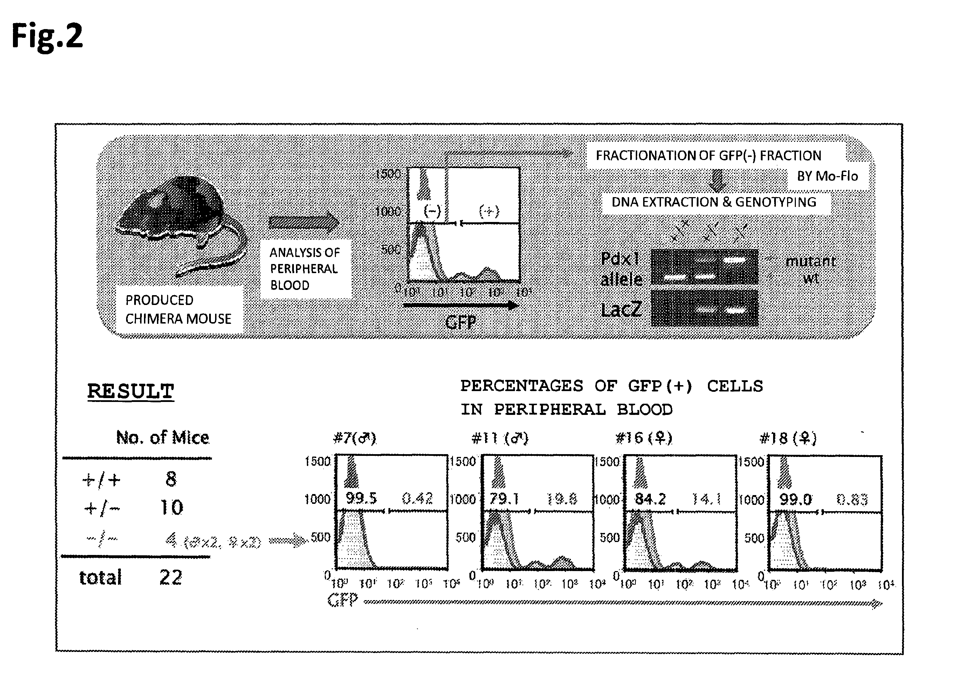

[Fig. 2] Fig. 2 shows that it was found from result of genotyping that the knockout mice with a pancreas complemented grew to be normal adults. The upper panel shows the genotyping using host-derived cells in peripheral blood. At the lower left, result of the genotyping is shown. The ratios of mice having been identified by this method are shown. In this data, there were 4 KO individuals which could become founders in a total of 22 mice. At the right, chimerism rates of respective individuals are shown. The chimerism rate was analyzed by indicating the percentage of GFP (+) cells in peripheral blood.

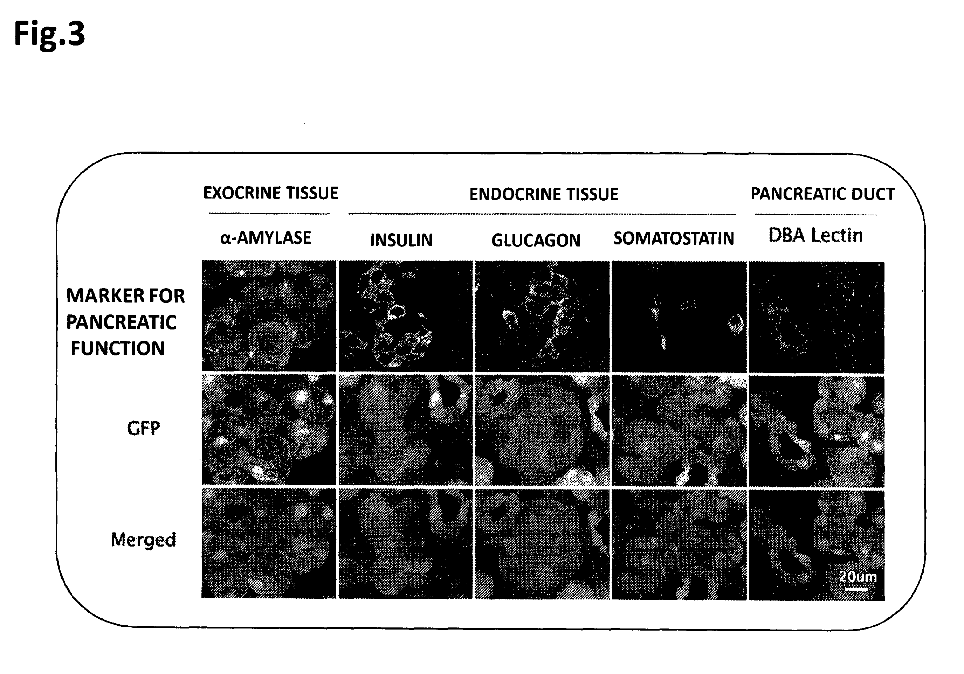

[Fig. 3] Fig. 3 shows that the ES cell-derived pancreas has a marker of pancreatic function being expressed in vivo (analysis was carried out in the neonatal period). From the top, a marker of pancreatic function, GFP, and merged view are shown. From the left in order, α-amylase which is a marker of exocrine tissue, and insulin, glucagon, and somatostatin, which are markers of endocrine tissue, are shown. On the right, DBA lectin, which is a marker of pancreatic duct, is shown.

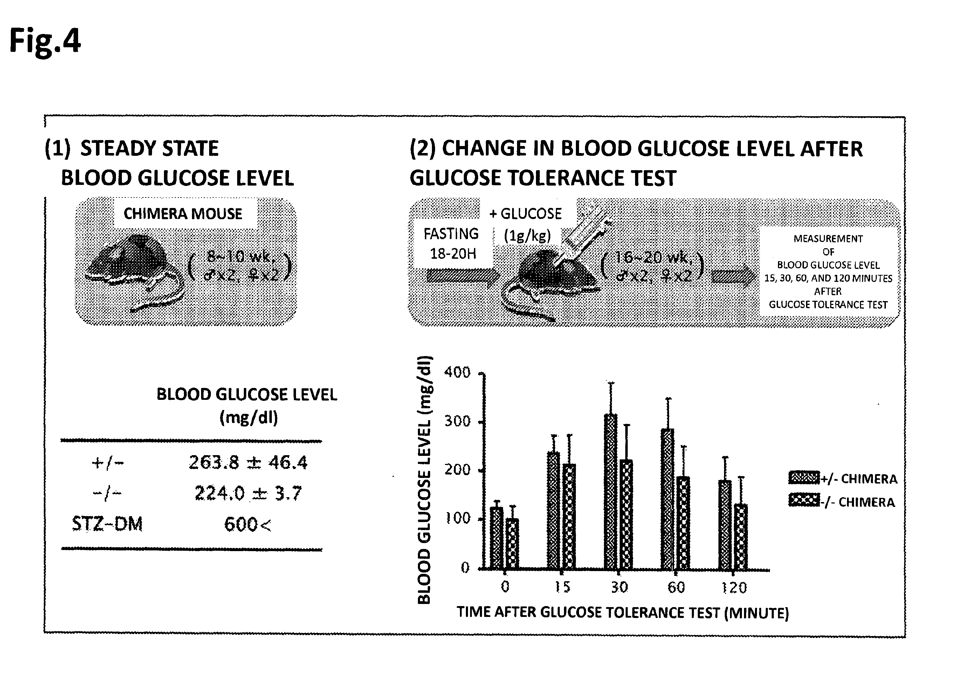

[Fig. 4] Fig. 4 shows that the mice with a pancreas complemented are also capable of controlling blood glucose level normally. On the left, steady state blood glucose levels are shown. On the right, changes in the blood glucose levels after a glucose tolerance test are shown. Blood glucose levels were measured 15, 30, 60, and 120 minutes after the glucose tolerance test.

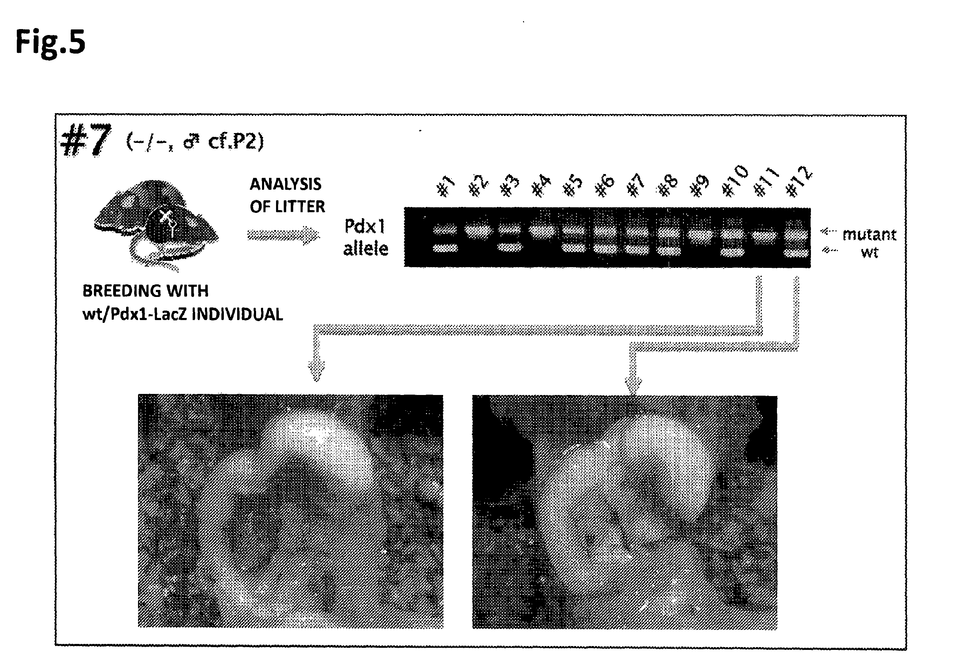

[Fig. 5] Fig. 5 shows that the knockout mice with a pancreas complemented by ES cells are capable of transmitting its phenotype to the next generation. As shown at the upper left, the knockout mice were each bred with a wt/Pdxl-LacZ individual. Analysis of Pdx1 allele by analyzing a resulting litter is shown on the right. On the lower part, photographs of actual dissection are shown.

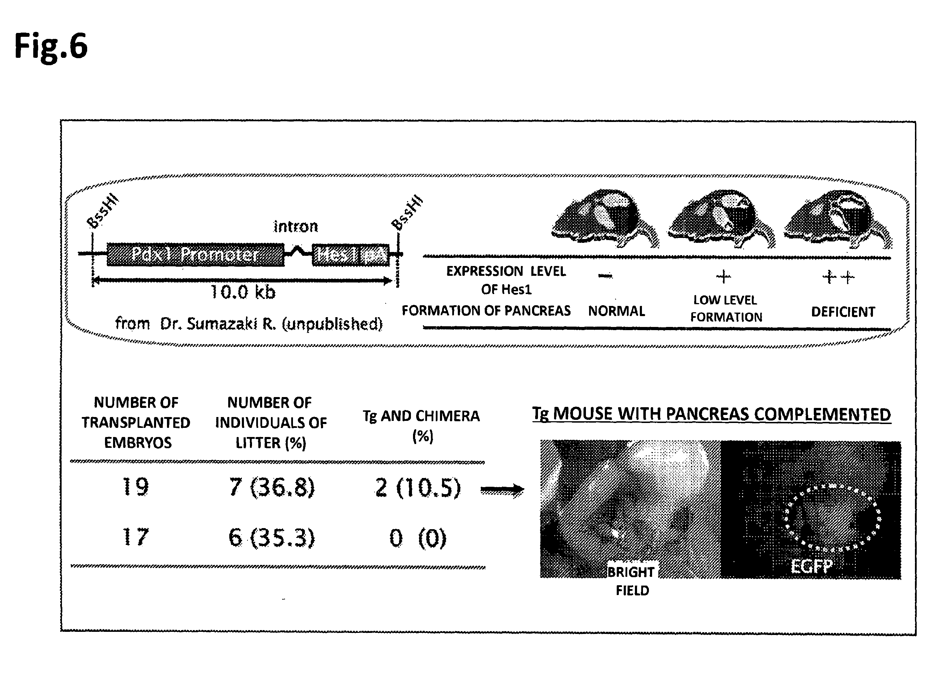

[Fig. 6] Fig. 6 shows regeneration of pancreas by blastocyst complementation in Pdx1-Hes1 transgenics. In the upper panel, a construction of an introduced vector is shown. On the right of the panel, identification criteria are described. The degree of formation of pancreas differs depending on the expression level of Hes1 which is expressed under the Pdx1 promoter (expressed especially in a fetal pancreas). When the level of expression is high (that is, the copy number is high), a deficiency of pancreas is indicated. On the right bottom, a transgenic mouse with a pancreas complemented is shown. It has been revealed that it is possible to create a pancreas derived from ES cell using the Pdx1-Hes1 transgenic mouse (the table on the left bottom shows the result).

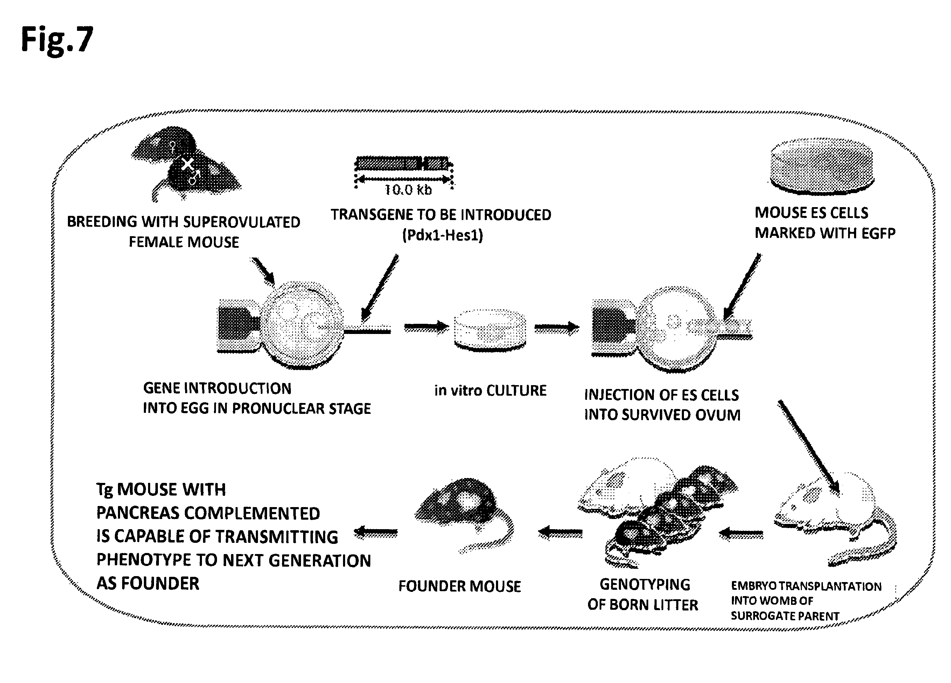

[Fig. 7] Fig. 7 shows a strategic scheme for producing a founder transgenic mouse on the basis of the combination of a technique for producing transgenics and blastocyst complementation. An egg cell is obtained by breeding with a female mouse having been subjected to ovarian superstimulation, and gene transfer into an egg in the pronuclear stage is performed (here, the transgene (Pdx1-Hes1) to be introduced), and then in vitro culture is carried out. A survived ovum is subjected to injection of ES cells, and then embryo transfer into a womb of a surrogate parent is performed. A born litter is subjected to genotyping, thereby creating a founder mouse. A transgenic mouse with a pancreas complemented is capable of transmitting the phenotype thereof to the next generation as a founder.

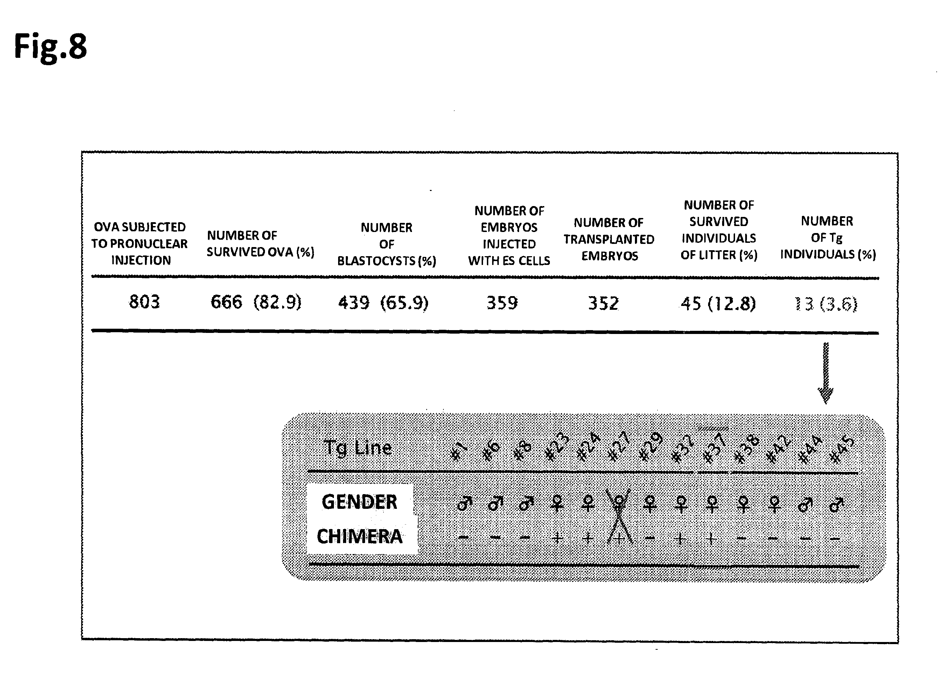

[Fig. 8] Fig. 8 shows an attempt for production of a founder transgenic mouse. From the left, ova subjected to pronuclear injection, the number of survived ova, the number of blastocysts, the number of embryos injected with ES cells, the number of transplanted embryos, the number of survived individuals of the litter, and the number of transgenic individuals, and, if necessary, the percentages thereof are shown. Chimeras were determined by the coat color. Since the donor ES cells are derived from the mouse strain 129 (agouti) and the host embryo is derived from C57BL6xBDF1 (black), it is possible to determine whether or not an individual is a chimera when its coat has fully grown.

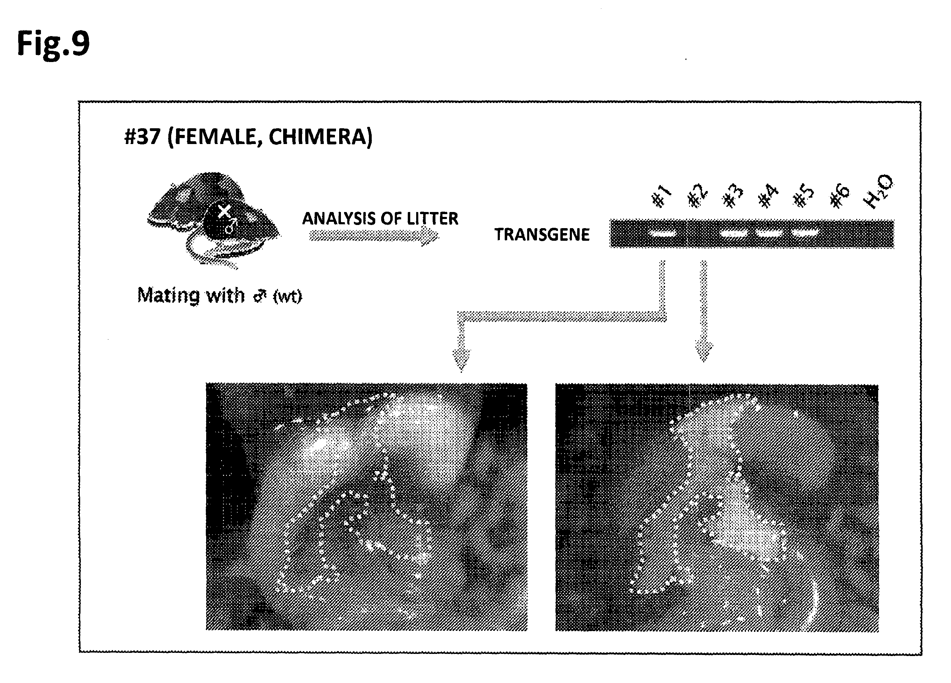

[Fig. 9] Fig. 9 shows that the transgenic mouse with a pancreas complemented is capable of transmitting its phenotype to the next generation. A produced transgenic chimeric individual is bred with a wild type. It is revealed whether the phenotype of pancreas deficiency is transmitted to the next generation, by morphological analysis of pancreases of a litter or by PCR on the genomic DNA. If the transgenic chimera can be a founder, a mouse lacking a pancreas should be born in the next generation. Here, it is shown that the mouse #37 can function as a founder. On the right, confirmation of the transgene in the analysis on the litter is shown, and the lower panel shows actual photographs. It is inferred that #37 showed normality after birth because a pancreas thereof was complemented during the production. As shown here, even with transgenic mice, it is possible to produce a founder which allows efficient production of mice, such as organ-deficient mice, which would die in the embryonic stage or immediately after birth.

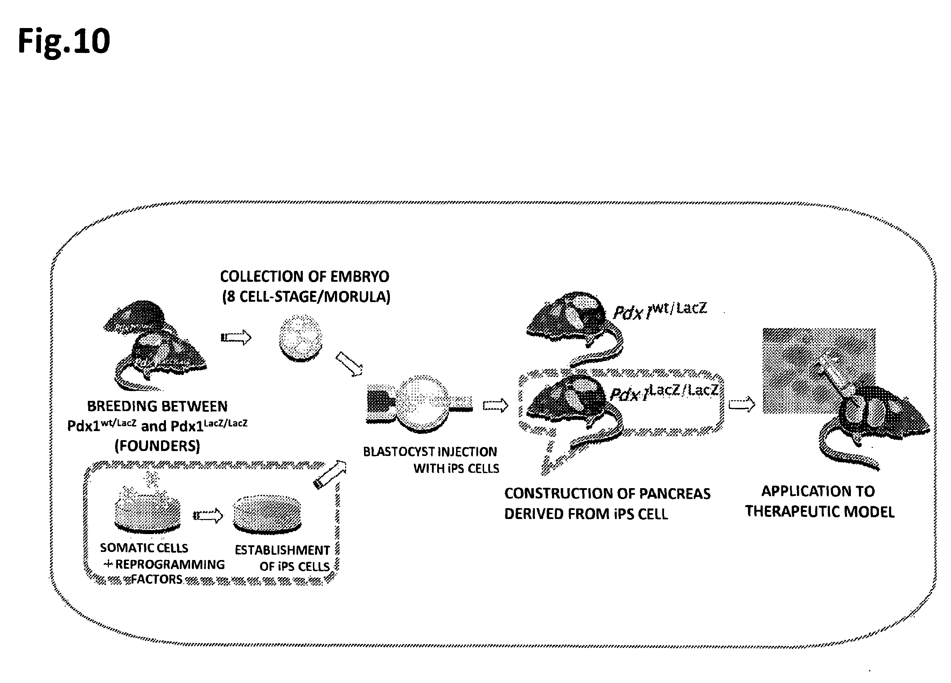

[Fig. 10] Fig. 10 shows a therapeutic model using the construction of a pancreas derived from iPS cell by blastocyst complementation.

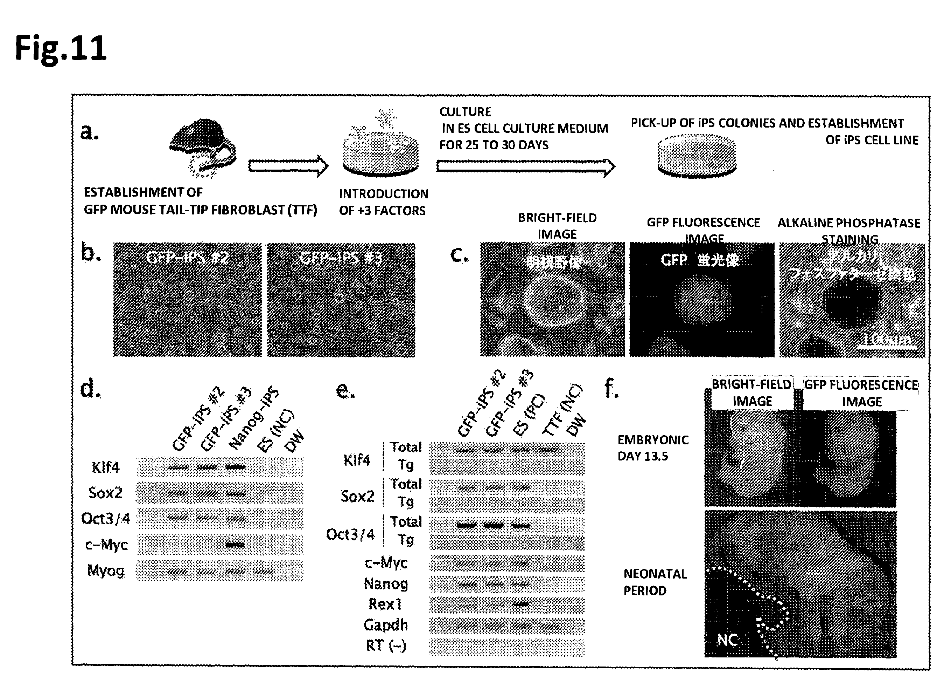

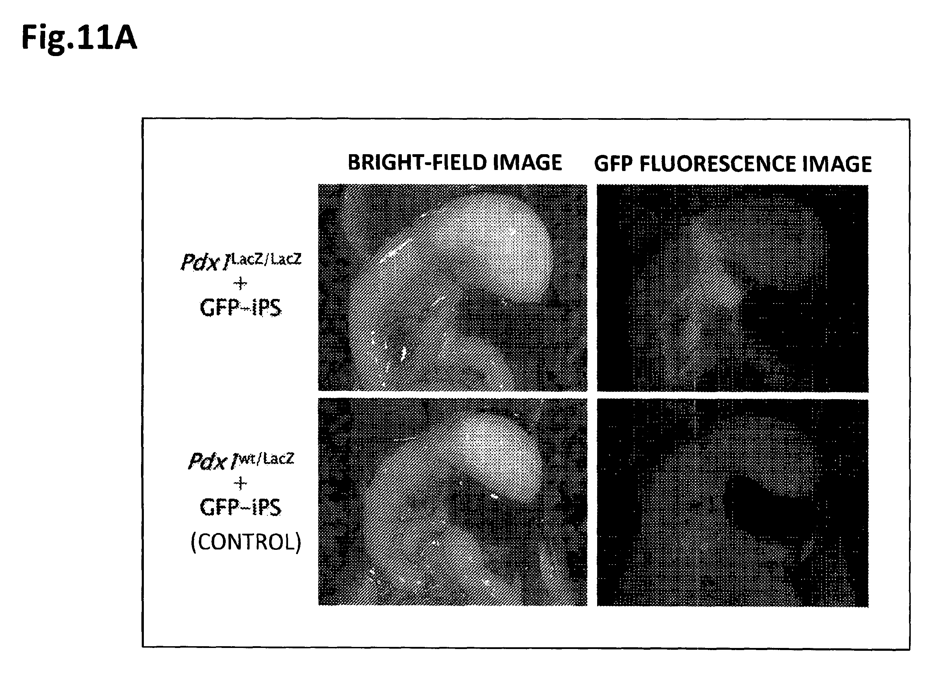

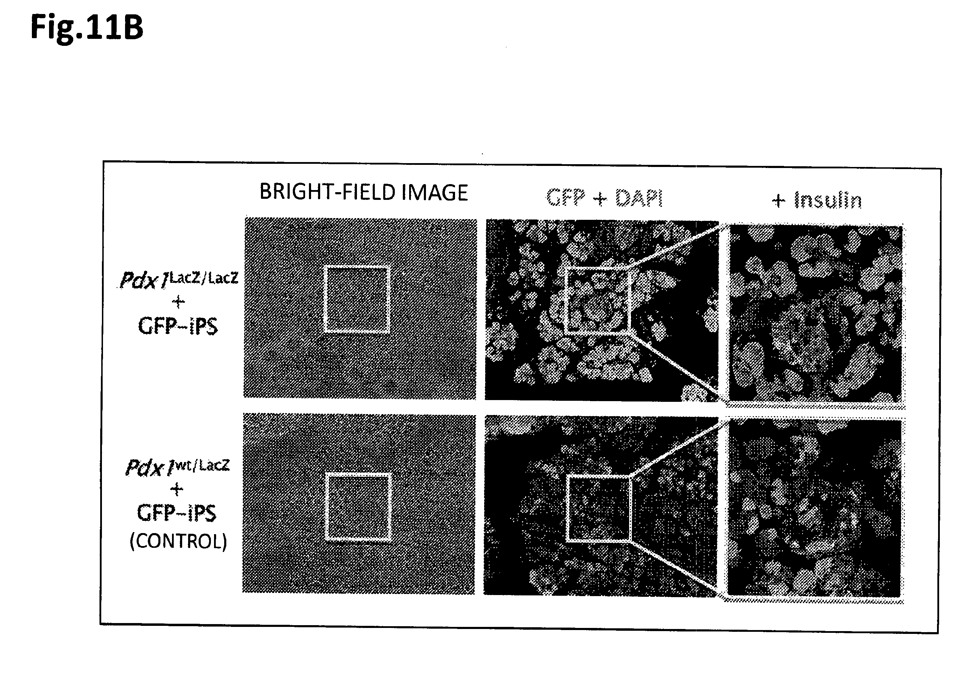

[Fig. 11] a. shows a strategy for establishing GFP mouse-derived iPS cells. After establishment of GFP mouse tail-tip fibroblasts (TTF), three factors (reprogramming factors) were introduced into the TTF, and resulting TTF was cultured in ES cell culture medium for 25 to 30 days. Then, iPS colonies were picked up, thereby establishing iPS cell lines. b. shows photographs of the morphology of thus established iPS cells taken by a microscope equipped with a camera. The left is a photograph of GFP-iPS cell #2, and the right is that of #3. c. shows measurements of alkaline phosphatase activity. The iPS cells were photographed under a fluorescent microscope, and subjected to staining using an alkaline phosphatase staining kit (Vector Laboratories, Inc., Cat. No. SK-5200). From the left, a bright-field image, a GFP fluorescence image, and alkaline phosphatase staining are shown. d. shows identification of the introduced three factors (reprogramming factors) by PCR on genomic DNA. It is the result obtained from PCR performed on the genomic DNA extracted from the iPS cells. From the top, expressions of Klf4, Sox2, Oct3/4, c-Myc, and Myog genes are shown. From the left, results of GFP-iPS cells #2 and #3, Nanog-iPS (that of four factors), and ES cell (NC) as a control are shown. At the very right, result of distilled water is shown. Insertion of the three factors in the iPS cells used in the present invention was confirmed. e. shows analysis of an ES cell-specific gene expression pattern in the cells used in the present invention and confirmation of the expression of the introduced genes, using RT-PCR. From the top, expressions of Klf4, Sox2, Oct3/4, c-Myc, Nanog, Rexl, and Gapdh genes are shown. At the bottom, a negative control (RT (-)) is shown. As for Klf4, Sox2, and Oct3/4, the expression was confirmed each for total RNA and transgenic (Tg) RNA. From the left, expressions of GFP-iPS cells #2 and #3, ES cell (NC) as a control, and TTF (negative control) as another control are shown. At the very right, result of distilled water is shown. f. shows production of a chimeric mouse using iPS cells. Result of the production of a chimeric mouse is shown, the production being performed by injecting the established iPS cells into a blastocyst obtained from breeding between C57BL6 and BDF1 mouse strains. In the upper part, a bright-field image (left) and a GFP fluorescence image (right) of the mouse at embryonic day 13.5 are shown. In the lower part, an image of the mouse in neonatal period is shown. What denoted by NC is a negative control.
[Fig. 11A] Fig. 11A shows morphologies of pancreases constructed by blastocyst complementation (5 days after birth). While the border of the pancreas of the homo mouse is neatly made up of GFP-positive cells, that of the pancreas of the hetero mouse is chimeric, which can be observed as a dotted line.
[Fig. 11B] Fig. 11B shows histological analysis of pancreases derived from iPS cell (5 days after birth). Here, frozen section samples of the pancreases derived from iPS cell were prepared, subjected to nuclear staining with DAPI, an anti-GFP antibody, and an anti-insulin antibody, and then observed and photographed using an upright fluorescence microscope and a confocal laser microscope. From the left, bright-light images and GFP+DAPI images are shown, and staining using the anti-insulin antibody is shown on the right. The upper panels show Pdx1^{LacZ/LacZ} of the present invention into which GFP-iPS cells had been introduced, and the lower panels show Pdx1^{wt/Lacz} as a control into which GFP-iPS cells had been introduced.

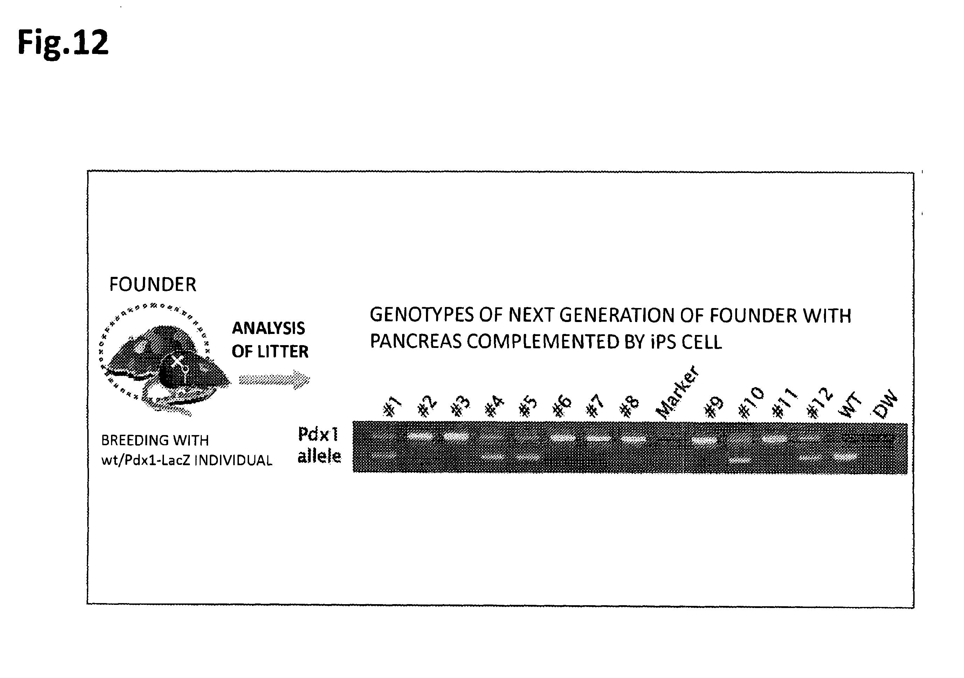

[Fig. 12] Fig. 12 shows that a knockout mouse with a pancreas complemented by iPS cells is capable of transmitting the phenotype as a founder to the next generation. As shown in the upper left, the knockout mouse was bred with a wt/Pdx1-LacZ individual. Analysis of Pdx1 allele by analyzing a resulting litter is shown on the right.

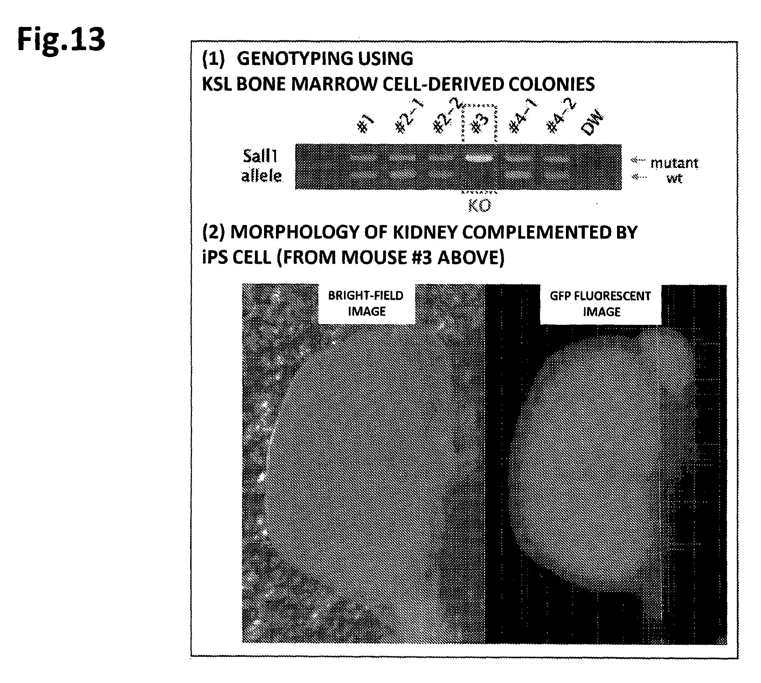

[Fig. 13] Fig. 13 shows regeneration of a kidney by blastocyst complementation in a Sall1 knockout mouse. Result from genotyping of the Sall1 allele is shown in the upper part. It is understood that the mouse #3 was a Sall1 homo KO mouse. On the lower part, morphology of the kidney (1 day after birth) regenerated by performing blastocyst complementation with iPS cells in the mouse #3 as a host is shown. It is shown that the whole kidney in the homo KO mouse is neatly made up of GFP-positive cells. It has been revealed that it is possible to produce a kidney derived from iPS cells using a Sall1 knockout mouse.

### Sequence Table Free Text

Sequence Number 1 Forward (Fw) primer for identification of cell derived from injected embryo: TTC ATG CGA CGG TTT TGG AAC
Sequence Number 2 Reverse 1 (Rv1) primer for identification of cell derived from injected embryo: TTC AAC ATC ACT GCC AGC TCC
Sequence Number 3 Reverse (Rv2) primer for identification of cell derived from injected embryo: TGT GAG CGA GTA ACA ACC
Sequence Number 4 Forward (Fw) primer for detection of transgene: TGA CTT TCT GTG CTC AGA GG
Sequence Number 5 Reverse (Rv) primer for detection of transgene: CAA TGA TGG CTC CAG GGT AA
Sequence Number 6 Fw primer for detection of Oct3/4 (mOct3/4-S1120): CCC TGG GGA TGC TGT GAG CCA AGG
Sequence Number 7 Rv primer for detection of Oct3/4 (pMX/L3205): CCC TTT TTC TGG AGA CTA AAT AAA
Sequence Number 8 Fw primer for detection of Klf4 (Klf4-S1236): GCG AAC TCA CAC AGG CGA GAA ACC
Sequence Number 9 Rv primer for detection of Klf4·Sox2·c-Myc (pMXs-AS3200): TTA TCG TCG ACC ACT GTG CTG CTG
Sequence Number 10 Fw primer for detection of Sox2 (Sox2-S768): GGT TAC CTC TTC CTC CCA CTC CAG
Sequence Number 11 FW primer for detection of c-Myc (c-Myc-S1093): CAG AGG AGG AAC GAG CTG AAG CGC
Sequence Number 12 Forward primer for detection of cell (mutant) derived from injected embryo: AAG GGA CTG GCT GCT ATT GG
Sequence Number 13 Forward primer for detection of cell (wild type) derived from injected embryo: GTA CAC GTT TCT CCT CAG GAC
Sequence Number 14 Reverse primer for cell (mutant) derived from injected embryo: ATA TCA CGG GAT GCC AAC GC
Sequence Number 15 Reverse primer for detection of cell (wild type) derived from injected embryo: TCT CCA GTG TGA GTT CTC TCG

### DETAILED DESCRIPTION OF THE PREFERRED EMBODIMENTS

Hereinafter, the present invention will be described. It should be understood throughout the present description that expression of a singular form includes the concept of its plurality unless otherwise mentioned. Accordingly, articles for a singular form (for example, "a," "an," "the," and the like, in English) also include the concept of their plurality unless otherwise mentioned. It should also be understood that the terms as used herein have definitions typically used in the art unless otherwise mentioned. Thus, unless otherwise defined, all technical terms and scientific terms as used herein have the same meanings as those generally understood by those skilled in the art to which the present invention pertain. If there is contradiction, the present description (inclusive of the definition) takes precedence.

The terms a "founder" creature and a "founder" animal as used herein refer to a creature and an animal, respectively, which allow next generation progenies and those coming after to survive as maintaining a desired phenotype (especially, deficiency of an organ or the like).

The term "giving no possibility of survival or difficulty in survival if it functions" as used herein refers to a condition, regarding a certain factor, in which, if the factor functions, an animal as a host cannot survive at all and die, or can survive but is substantially impossible to survive thereafter due to reasons, such as difficulties in growth and reproduction. This term can be understood by using ordinary knowledge in the art.

As used herein, the term "an organ or body part, which gives no possibility of survival or difficulty in survival, if it functions" refers to an organ or body part, regarding a certain factor, which gives no possibility of survival or difficulty in survival when the factor causes a deficiency or dysfunction (for example, not normal) of the organ or body part. For example, in the case of a foreign gene, when the gene is introduced into a creature and expressed normally, a deficiency occurs in a certain organ or body part, resulting in the creature being incapable of survival or having difficulty in survival. Difficulty in survival includes incapability of procreation of the next generation. Such an organ or body part may be, for example, pancreas, kidney, and the like, but is not limited thereto.

As used herein, the terms "(deficiency responsible) gene coding for a factor which causes a deficiency of an organ or body part and gives no possibility of survival or difficulty in survival if the factor functions" and "deficiency responsible gene" are used interchangeably. When referring to a certain gene, these terms refer to a gene which causes a deficiency or dysfunction of an organ or body part when the factor functions (for example, by being introduced to be expressed in the case of a foreign gene, or by being exposed to a condition in which such a gene functions in the case of an intrinsic gene), thereby giving no possibility of survival or difficulty in survival.

The term "organ" as used herein is used to have an ordinary meaning in the art, and refers to organa constituting animal viscera in general.

The term "body part" as used herein refers to any part of a body, and also includes ones which are not generally referred to as organs. For example, when a kidney is taken as an example, a complete kidney is created when genes are normal. However, when some gene is deficient or has abnormality, although an organ like a kidney may be created, a part of the organ may have abnormality or deficiency. The part having such an abnormality or deficiency can be said to be an example of this "body part." Gene deficiency or abnormality does not necessarily correspond to each organ, and it frequently occurs that a part thereof is affected. Accordingly, when a correspondence relationship to a gene is to be considered, it may be better to consider correspondence to a body part. Therefore, such a correspondence relationship is also taken into consideration herein.

The term "blastocyst complementation" as used herein refers to a technique for complementing a defective organ or body part by using the phenomenon in which a resulting individual obtained from transplantation of pluripotent stem cells, such as ES cells and iPS cells, having multipotency into an inner space of a blastocyst stage fertilized egg forms a chimeric mouse. The basic procedures of such a technique can be referred to Patent Document 1 or the like.

The term "label" as used herein refers to a factor used for distinguishing a complemented organ from a host. Accordingly, only an organ or only a host may be labeled, or both may be labeled with clearly distinguishable labels, respectively.

The term "first pluripotent stem cell" as used herein refers to a pluripotent cell used as an origin to be a host of a founder animal or a cell mass derived from the pluripotent stem cell, and a fertilized egg·embryo are preferably used.

The term "second pluripotent stem cell" as used herein refers to a pluripotent stem cell which is used with a view of an organ to be produced.

The term "being capable of complementing a deficiency" as used herein refers to an ability to complement an organ or body part, when a factor, gene, or the like is referred.

The term "chimeric blastocyst" as used herein refers to a blastocyst formed by a cell, which is derived from the first pluripotent stem cell, and a cell, which is derived from the second pluripotent stem cell, being in a chimeric state.

### (Molecular Biology)

The terms "protein," "polypeptide," "oligopeptide" and "peptide" as used herein have the same meaning herein and refer to an amino acid polymer having any length. This polymer may be a straight-chained, branched, or cyclic polymer. An amino acid may be a naturally occurring or non-naturally occurring amino acid, or a variant amino acid. These terms may also include those assembled into a composite of multiple polypeptide chains. These terms also includes naturally occurring or artificially modified amino acid polymers. Examples of such modification include: disulfide bond formation; glycosylation; lipidation; acetylation; phosphorylation; and any other manipulation and modification (for example, conjugation with a labeling moiety). This definition also includes polypeptides containing at least one amino acid analog (for example, containing non-naturally occurring amino acids and the like), peptide-like compounds (for example, peptoids), and other variants known in the art, for example.

As used herein, the term "amino acid" may be a naturally occurring or non-naturally occurring amino acid as long as it satisfies the purpose of the present invention.

The term "nucleic acid" as used herein is used interchangeably with gene, cDNA, mRNA, oligonucleotide, and polynucleotide. A particular nucleic acid sequence also includes "splice variants." Similarly, a particular protein encoded by a nucleic acid implicitly includes any protein encoded by a splice variant of that nucleic acid. "Splice variants," as the name suggests, are products of alternative splicing of a gene. After transcription, an initial nucleic acid transcript may be spliced such that different (alternative) nucleic acid splice products encode different polypeptides. Mechanisms for the production of splice variants vary, but also include alternative splicing of exons. Other polypeptides derived from the same nucleic acid by read-through transcription are also included by this definition. Any products of a splicing reaction (including recombinant forms of the splice products), are included in this definition. Alternatively, allelic mutants are also included in this range.

As used herein, the terms "polynucleotide," "oligonucleotide," and "nucleic acid" have the same meaning herein and refer to a nucleotide polymer having any length. These terms also include an "oligonucleotide derivative" or a "polynucleotide derivative." An "oligonucleotide derivative" or a "polynucleotide derivative" includes a nucleotide derivative, or refers to an oligonucleotide or a polynucleotide having different linkages between nucleotides from typical linkages, and are used interchangeably. Examples of such oligonucleotides specifically include: 2'-O-methyl-ribonucleotide; an oligonucleotide derivative in which a phosphodiester bond in an oligonucleotide is converted to a phosphorothioate bond; an oligonucleotide derivative in which a phosphodiester bond in an oligonucleotide is converted to a N3'-P5' phosphoroamidate bond; an oligonucleotide derivative in which ribose and a phosphodiester bond in an oligonucleotide are converted to a peptide-nucleic acid bond; an oligonucleotide derivative in which uracil in an oligonucleotide is substituted with C-5 propynyl uracil; an oligonucleotide derivative in which uracil in an oligonucleotide is substituted with C-5 thiazole uracil; an oligonucleotide derivative in which cytosine in an oligonucleotide is substituted with C-5 propynyl cytosine; an oligonucleotide derivative in which cytosine in an oligonucleotide is substituted with phenoxazine-modified cytosine; an oligonucleotide derivative in which ribose in DNA is substituted with 2'-O-propyl ribose; an oligonucleotide derivative in which ribose in an oligonucleotide is substituted with 2'-methoxyethoxy ribose; and the like. Unless otherwise indicated, particular nucleic acid sequences are also intended to include conservatively-modified variants thereof (for example, degenerate codon substitutions) and complementary sequences as well as sequences explicitly indicated. Specifically, degenerate codon substitutions may be produced by generating sequences in which the third positions of one or more selected (or all) codons are substituted with mixed-bases and/or deoxyinosine residues (Batzer et al. , Nucleic Acid Res. 19: 5081 (1991) ; Ohtsuka et al., J. Biol. Chem. 260: 2605-2608 (1985); Rossolini et al., Mol. Cell. Probes 8: 91-98 (1994)).

The term "nucleotide" as used herein may be a naturally occurring or non-naturally occurring nucleotide as long as a target function can be maintained.

The term "search" as used herein indicates that a given nucleic acid sequence is utilized to find other nucleic acid base sequences having a specific function and/or property either electronically or biologically, or using other methods. Examples of the electronic search include, but are not limited to: BLAST (Altschul et al., J. Mol. Biol. 215: 403-410 (1990)); FASTA (Pearson & Lipman, Proc. Natl. Acad. Sci., USA 85: 2444-2448 (1988)); Smith and Waterman method (Smith and Waterman, J. Mol. Biol. 147: 195-197 (1981)); Needleman and Wunsch method (Needleman and Wunsch, J. Mol. Biol. 48: 443-453 (1970)); and the like. Examples of the biological search include, but are not limited to: stringent hybridization; a macroarray in which genomic DNA is attached to a nylon membrane or the like and a microarray (microarray assay) in which genomic DNA is attached to a glass plate; PCR and in situ hybridization; and the like. In the present description, it is intended that a corresponding gene identified by the aforementioned electronic search or biological search should also be included in the genes (for example, Sall1, Pdx-1, and the like) used in the present invention.

A nucleic acid sequence hybridizing with a specific gene sequence can also be used herein as long as it has a function. As used herein, the term "stringent conditions for hybridization" refers to conditions that a complementary chain of a nucleotide strand having similarity or homology with respect to a target sequence hybridizes preferentially with the target sequence and a complementary chain of a nucleotide strand not having similarity or homology does not substantially hybridize. The "complementary chain" of a certain nucleic acid sequence indicates a nucleic acid sequence pairing therewith on the basis of hydrogen bonds between bases of a nucleic acid (for example, T to A, and C to G). The stringent conditions are sequence-dependent, and vary under various circumstances. A longer sequence specifically hybridizes at higher temperature. Generally, the stringent conditions are selected to be approximately 5°C lower than the thermal melting point (Tm) of a specific sequence at a defined ionic strength and pH. Tₘ is a temperature at which 50% of a nucleotide complementary to a target sequence hybridizes with the target sequence in an equilibrium state under a defined ionic strength, pH, and nucleic acid concentration. The "stringent conditions" are sequence-dependent, and vary depending on various environmental parameters. General guidelines for nucleic acid hybridization are found in Tijssen (Tijssen (1993), Laboratory Techniques In Biochemistry And Molecular Biology - Hybridization With Nucleic Acid Probes Part I, Chapter 2, "Overview of principles of hybridization and the strategy of nucleic acid probe assay," Elsevier, N.Y.).

Typically, the stringent conditions are conditions in which a salt concentration is less than approximately 0.1 M Na⁺, and typically a concentration of approximately 0.01 to 1.0 M Na⁺ (or other salt) at pH 7.0 to 8.3, and a temperature is at least approximately 30°C for short nucleotide sequences (for example, 10 to 50 nucleotides), and at least approximately 60°C for long nucleotide sequences (for example, longer than 50 nucleotides). The stringent conditions also can be achieved by adding a destabilizing agent, such as formamide. Examples of the stringent conditions according to the present description include: hybridization performed in a buffer solution containing 50% formamide, 1M NaCl, and 1% SDS (37°C); and washing with 0.1×SSC at 60°C.

The term "polynucleotide hybridized under stringent conditions" as used herein refers to well-known conditions that are commonly used in the art. Such a polynucleotide may be obtained by a colony hybridization method, a plaque hybridization method, a Southern blotting hybridization method, or the like, using a polynucleotide selected from the polynucleotides of the present invention as a probe. Specifically, such a polynucleotide may be identified by hybridization using a filter, on which a DNA derived from a colony or a plaque is immobilized, in the presence of 0.7 to 1.0 M NaCl at 65°C, followed by washing the filter with SSC (saline-sodium citrate) solution having 0.1- to 2-fold concentration (SSC solution at a 1-fold concentration contains 150 mM sodium chloride and 15 mM sodium citrate) at 65°C. Hybridization may be conducted according to the method described in experimental manuals, such as Molecular Cloning 2nd ed., Current Protocols in Molecular Biology, Supplement 1-38, DNA Cloning 1: Core Techniques, A Practical Approach, Second Edition, Oxford University Press (1995). Preferably, sequences hybridized under stringent conditions herein exclude those sequences containing an A sequence only or a T sequence only. The term "hybridizable polynucleotide" as used herein refers to a polynucleotide which can hybridize with another polynucleotide under the above-described hybridization conditions. Specific examples of the hybridizable polynucleotide include a polynucleotide having at least 60% homology with the base sequence of a DNA encoding a polypeptide having an amino acid sequence that is specifically shown in the present invention, preferably a polynucleotide having at least 80% homology and a polynucleotide having at least 90% homology, and more preferably a polynucleotide having at least 95% homology.

An amino acid can be referred to herein by either the generally known three-letter symbol, or the one-letter symbol recommended by the IUPAC-IUB Biochemical Nomenclature Commission. Likewise, a nucleotide can also be referred to by the generally-accepted one-letter code.

The term "homology" of a gene as used herein refers to the degree of identity of two or more gene sequences with each other. Therefore, the higher the homology between two certain genes, the higher the identity or similarity between their sequences. Whether or not two genes have homology may be determined by comparing their sequences directly, or, in the case of a nucleic acid, by a hybridization method under stringent conditions. When two gene sequences are directly compared with each other, the genes have homology if the DNA sequences of the gene sequences are typically at least 50% identical, preferably at least 70% identical, more preferably at least 80%, 90%, 95%, 96%, 97%, 98%, or 99% identical.

In the present description, comparisons of similarity, identity, and homology between amino acid sequences and base sequences are calculated using BLAST, which is a tool for sequence analysis, and using default parameters. A search for identity may be performed using, for example, BLAST 2.2.9 (published on May 12, 2004) of NCBI. A value of identity herein is usually a value obtained from alignment carried out using the above-mentioned BLAST under the default conditions. However, if a higher value is obtained as a result of a change in parameter, the highest value will be designated as a value of identity. If identity is evaluated in multiple domains, the highest value among resulting values is designated as a value of identity.

As used herein, the term "corresponding" gene refers to a gene, in a certain species, which has, or is anticipated to have, an action similar to that of a predetermined gene in a species as a reference for comparison. If there are multiple genes each having such an action, the term refers to a gene having the same evolutionary origin. Therefore, a gene corresponding to a certain gene (for example, Sall1 or Pdx-1) may be an orthologue of the certain gene. Therefore, genes corresponding to human genes may be found in other animals (mouse, rat, pig, rabbit, guinea pig, cattle, sheep, and the like) as well. Such corresponding genes may be identified using a technique that is well known in the art. Therefore, for example, a corresponding gene in a certain animal may be found by searching a sequence database of the animal (for example, mouse, rat, pig, rabbit, guinea pig, cattle, sheep, and the like), using the sequence of a gene, that serves as the reference for the corresponding gene, as a query sequence.

The term "fragment" as used herein refers to a polypeptide or a polynucleotide having a sequence length ranging from 1 to n-1, with respect to a full-length polypeptide or polynucleotide (having a length of n). The length of the fragment may be appropriately changed in accordance with the purpose, and for example, in the case of a polypeptide, the lower limit of the length thereof may be 3, 4, 5, 6, 7, 8, 9, 10, 15, 20, 25, 30, 40, 50 and more amino acids. Lengths that are represented by integers but are not specified herein (for example, 11 and the like) may also be appropriate as the lower limit. Further, in the case of a polynucleotide, the lower limit of the length thereof may be 5, 6, 7, 8, 9, 10, 15, 20, 25, 30, 40, 50, 75, 100 and more nucleotides. Lengths that are represented by integers but are not specified herein (for example, 11 and the like) may also be appropriate as the lower limit. In the present description, the lengths of a polypeptide and a polynucleotide may be represented by the number of amino acids or nucleic acids. However, the numbers mentioned above are not intended to be absolute, and the numbers as the upper or lower limit are intended to also include some numbers above and below the numbers (or, for example, above and below by 10%), as long as the same function is maintained. For the purpose of expressing such intention, the term "approximately" may be attached before the number herein. However, it should be understood that the presence or absence of "approximately" herein does not affect the interpretation of the number. A useful length of a fragment herein may be determined based on whether or not at least one function is maintained among the functions of the full-length protein which serves as the reference for the fragment.

As used herein, the term "variant" refers to a substance, such as a polypeptide and a polynucleotide, having been partially modified as compared to the original substance. Examples of such a variant include: a substitution variant; an addition variant; a deletion variant; a truncated variant; an allelic mutant; and the like. The term "allele" refers to gene variants that belong to the same genetic locus, and are discriminated from each other. Therefore, the term "allelic mutant" refers to a variant that is in the relationship of allele with respect to a certain gene. The term "homolog" refers to a sequence having homology (preferably at least 60% homology, more preferably at least 80%, at least 85%, at least 90%, or at least 95% homology) with a certain gene within a certain species at the amino acid or nucleotide level. The method of obtaining such a homolog is apparent from the description herein.

In the present description, in order to produce a functionally equivalent polypeptide, addition, deletion, or modification of amino acid can also be carried out in addition to substitution of amino acid. The substitution of amino acid means substituting an original peptide with one or more, for example, 1 to 10, preferably 1 to 5, and more preferably 1 to 3, amino acids. The addition of amino acid means adding one or more, for example, 1 to 10, preferably 1 to 5, and more preferably 1 to 3, amino acids to an original peptide chain. The deletion of amino acid means deletion of one or more, for example, 1 to 10, preferably 1 to 5, and more preferably 1 to 3, amino acids from an original peptide. The modification of amino acid includes: amidation; carboxylation; sulfation; halogenation; alkylation; phosphorylation; hydroxylation; acylation (for example, acetylation); and the like, but is not limited thereto. An amino acid to be substituted or added may be a naturally occurring amino acid, a non-naturally occurring amino acid, or an amino acid analogue. A naturally occurring amino acid is preferable.

These nucleic acids may be obtained by a publicly-known PCR method and may also be chemically synthesized. To these methods, for example, a site-directed mutagenesis method and a hybridization method may be combined.

As used herein, the term "substitution, addition, and/or deletion" of a polypeptide or polynucleotide refers to substitution, addition, or removal of an amino acid or a substitute thereof, or a nucleotide or a substitute thereof, in an original polypeptide or polynucleotide, respectively. Techniques for these substitution, addition and/or deletion are known in the art, and examples of the techniques include a site-directed mutagenesis and the like. These changes in a reference nucleic acid or polypeptide may occur at the 5'-terminal or 3'-terminal of this nucleic acid, or may occur at the amino terminal site or the carboxy terminal site of the amino acid sequence representing this polypeptide, or may occur at any site between those terminal sites so that the changes are present individually between residues of the reference sequence, as long as a desired function (for example, deficiency of kidney, deficiency of pancreas, or the like) is maintained. The substitution, addition, or deletion may occur in any number of times as long as it is once or more, and such a change may occur many times, as long as a desired function (for example, deficiency of kidney, deficiency of pancreas, or the like) is maintained. For example, the number of such a change may be one or several, and preferably up to 20%, up to 15%, up to 10% or up to 5% of the total length, or 150 or less, 100 or less, 50 or less, 25 or less, or the like.

In order to describe embodiments of the present invention specifically, exemplary embodiments will be described hereinafter.

### (Method for Producing Founder Animal for Reproduction)

One aspect of the present invention relates to an animal, which has a deficiency responsible gene coding for a factor which causes a deficiency of an organ or a body part and gives no possibility of survival or difficulty in survival if the factor functions, and in which the organ or the body part is complemented by blastocyst complementation.

By producing a next generation animal using this animal (also referred to as a "founder animal" herein), it is possible to cause a target organ to be deficient, and to produce an organ having a desired genome type regarding the deficient organ. Moreover, it has been revealed that production using this method enables organ production as well in the next generation. Thus, there has been a big breakthrough in industrial application of the present invention.

For a "organ or body part, which gives no possibility of survival or difficulty in survival, if it functions" used in a founder animal of the present invention, in regard to a certain factor, any organ or body part can be used as long as it gives no possibility of survival or difficulty in survival when the factor causes the organ or body part to be deficient or dysfunctional (for example, to be not normal). For example, in the case of a foreign gene, when the gene is introduced into an animal and expressed normally, a deficiency occurs in a certain organ or body part, resulting in the animal being incapable of survival or having difficulty in survival. Difficulty in survival includes incapability of procreation of the next generation. Such an organ or body part may be, for example, pancreas, kidney, or the like, but is not limited thereto.

Examples of genes involved in such events include Pdx-1 (for pancreas), Sall1 (for kidney), and the like.

Incidentally, to be used for organ regeneration, a gene should be selected with which an organ can be complemented and a resulting litter do not die after birth due to other factors (being incapable of ingesting milk from a mother mouse). One example of such a gene is Pdx-1. By using a gene possessing such properties, the invention of the present application can be carried out. In addition, even with the same phenotype of, for example, pancreatic deficiency, significance largely varies. Specifically, a knockout individual has a feature of improving productivity, while a transgenic individual has a feature of enabling clonal analysis of a lethal phenotype in addition to the feature of improving productivity.

The term "giving no possibility of survival or difficulty in survival if it functions" as used herein refers to a condition, regarding a certain factor, in which, if the factor functions, an animal as a host cannot survive at all and die, or can survive but is substantially impossible to survive later due to reasons, such as difficulties in growth and reproduction, and the term can be understood by using ordinary knowledge in the art. Such example, other than one originally having an organ or the like having such genetic traits, can be produced by introducing a gene possessing such traits.

The term "organ" as used herein is used to have an ordinary meaning in the art, and refers to organa constituting animal viscera in general. For a founder animal, especially, organs, such as kidney and pancreas, without which no next generation can exist, are assumed; however, it is understood that other than these are included in the range of the present invention.

The term "body part" as used herein refers to any part of a body, and also includes ones which are not generally referred to as organs. For example, when a kidney is taken as an example, a complete kidney is created when genes are normal. However, when some gene is deficient or has abnormality, although an organ like a kidney may be created, a part of the organ may have abnormality or deficiency. The part having such an abnormality or deficiency can be said to be an example of this "body part." Gene deficiencyor abnormality does not necessarily correspond to each organ, and it frequently occurs that a part thereof is affected. Accordingly, when a correspondence relationship to a gene is to be considered, it may be better to consider correspondence to a body part. Therefore, such a correspondence relationship is also taken into consideration herein. For example, a body part may be the island of Langerhans, which is a part of pancreas, or the glomerular, which is a part of kidney, but is not limited thereto.

The term "blastocyst complementation" as used herein refers to a technique for complementing a defective organ or body part by using the phenomenon in which a resulting individual obtained from transplantation of pluripotent stem cells, such as ES cells, having multipotency into an inner space of a blastocyst stage fertilized egg forms a chimeric mouse. The inventors have discovered, regarding blastocyst complementation which had been considered to be difficult, that a mammalian organ having a complicated cellular constitution formed from multiple kinds of cells, such as kidney, pancreas, hair, and thymus, can be produced in the living body of an animal, particularly, a non-human animal (Patent Document 1), and this technique can be used in full scale in the present invention.

The term "label" as used herein may be any factor as long as it is used for distinguishing a complemented organ from a host. Accordingly, only an organ or only a host may be labeled, or both may be labeled with clearly distinguishable labels, respectively. For example, by causing a specific gene (for example, a gene for expressing a fluorescence protein or the like) to be expressed only in an organ to be complemented, the organ to be complemented can be distinguished from a host of complementation by a property (for example, fluorescence) derived from the specific gene. Alternatively, by labeling only a host in a similar way, cells corresponding to a complemented organ can be distinguished by having a different label or no label. As described above, it can be distinguished whether an animal became complete by complementation with cells derived from exogenous cells or an animal became complete by complementation with cells derived from endogenous cells. Thus, it is possible to select a founder animal of the present invention more easily. These cells may incorporate, prior to transplantation, a fluorescent protein used for specific detection in an expressible state. For example, as a fluorescent protein used for such detection, the sequence of DsRed. T4 (Bevis B. J. and Glick B. S., Nature Biotechnology Vol.20, p. 83-87, 2002), which is a DsRed genetic mutant, may be designed so as to be expressed in organs of the almost entire body under the control of a CAG promoter (cytomegalovirus enhancer and chicken actin gene promoter), and then be incorporated into pluripotent stem cells, such as ES cells and iPS cells, by electroporation. By performing a fluorescent labeling on these cells for transplantation, it can be easily detected whether or not a produced organ is composed of transplanted cells only.

Examples of such label include, in addition to the above-described ones: green fluorescent protein (GFP) genes; red fluorescent proteins (RFP); cyan fluorescent proteins (CFP) ; other fluorescent proteins; LacZ; and the like.

In another aspect, the present invention provides a method for producing the animal of the present invention comprises the steps of: A) providing a first pluripotent stem cell having the deficiency responsible gene; B) growing the first pluripotent stem cell into a blastocyst; C) introducing a second pluripotent stem cell into the blastocyst so as to produce a chimeric blastocyst, the second pluripotent stem cell having an ability to complement a deficiency caused by the deficiency responsible gene; and D) producing individuals from the chimeric blastocyst, and then selecting an individual in which the any one of an organ and a body part has been complemented by the second pluripotent stem cell.

For a " (deficiency responsible) gene which codes for a factor which causes a deficiency of an organ or body part and gives no possibility of survival or difficulty in survival if the factor functions" (or a "deficiency responsible gene") used in the method for producing a founder animal of the present invention, any gene can be used as long as it causes a organ or body part to be deficient or dysfunctional (for example, to be not normal) of when the factor functions (for example, by being introduced to be expressed in the case of a foreign gene, or by being exposed to a condition in which such a gene functions in the case of an intrinsic gene), thereby giving no possibility of survival or difficulty in survival. Representative examples of such gene include Pdx-1 (for pancreas), Sall1 (for kidney), and the like.

Examples of "pluripotent stem cell" used in the present invention include: an egg cell; an embryonic stem cell (ES cell); an induced pluripotent stem cell (iPS cell); a multipotent germ stem cell (mGS cell); and the like. This is because the principle of production of a founder animal can be applied to other pluripotent stem cells once proved with ES cells. In fact, in the present invention, success has been proved in a method for producing a founder animal with iPS cells by using a similar technique and a method for producing an organ by using the founder animal.

As the "first pluripotent stem cell" used for a founder animal cell of the present invention, any cell or the like may be used as long as it is a pluripotent stem cell used as an origin to be a host of a founder animal or a cell mass derived from the pluripotent stem cell. Preferably, an inner cell mass (ICM), a fertilized egg, an embryo, or the like may be used.

The "second pluripotent stem cell" used in the present invention is a pluripotent stem cell used with a view of a organ to be produced, and may be any cell as long as it can grow as forming a chimeric blastocyst with the first pluripotent stem cell. For example, pluripotent stem cells, such as an ES cell and an iPS cell, can be used.

It is understood that any publicly-known one may be used for the "factor," "gene," or the like having an "ability of complementing a deficiency" as long as it is a factor, a gene, or the like, which is capable of complementing an organ or body part.

The "chimeric blastocyst" used in the present invention refers to a blastocyst formed by a cell, which is derived from the first pluripotent stem cell, and a cell, which is derived from the second pluripotent stem cell, being in a chimeric state. For example, the "chimeric blastocyst" can be produced by, in addition to an injection method, utilizing a so-called "aggregation method" in which embryo + embryo, or embryo + cell are closely attached to each other in a Petri dish to produce a chimeric blastocyst. Further, the relationship between a recipient embryo and a cell to be transplanted in the present invention may be a homologous relationship or a heterologous relationship. There have been hitherto a large number of reports on the preparation of a chimeric animal in such a heterologous relationship in the art, and, for example, there have been actually reported about blastular chimeric animals between closely related animal species, such as the preparation of a chimera between rat and mouse (Mulnard, J. G., C. R. Acad. Sci. Paris. 276, 379-381 (1973) ; Stern, M. S., Nature. 243, 472-473 (1973) ; Tachi, S. & Tachi, C. Dev. Biol. 80, 18-27 (1980); Zeilmarker, G., Nature, 242, 115-116 (1973)), and the preparation of a chimera between goat and sheep (Fehilly, C. B., et al. , Nature, 307, 634-636 (1984)). Therefore, in the present invention, in the case of preparing a kidney derived from cells of a mammal other than human in the living body of a mouse, a certain heterologous organ may be prepared in a recipient embryo based on these conventionally-known chimera creation methods (for example, a method of inserting cells to be transplanted into a recipient blastocyst (Fehilly, C. B. , et al. , Nature, 307, 634-636 (1984))).

The step of providing the first pluripotent stem cell having a gene, which codes for a factor which causes a deficiency of an organ or body part and gives no possibility of survival or difficulty in survival if the factor functions, (or a deficiency responsible gene) in the method for producing a founder animal of the present invention can be carried out, for example, by procuring a pluripotent stem cell having the gene or by producing a pluripotent stem cell having the gene by introducing the gene into a pluripotent stem cell. A method of such gene introduction is known in the art, and those skilled in the art can carry out such gene introduction by appropriately selecting a method. It is preferable to use electroporation. In electroporation, an electric pulse is applied to a cell suspension to create fine pores on a cell membrane, and DNA is sent into the cell so that transformation, that is, introduction of a target gene can be achieved. Accordingly, damage after electroporation is small. This is why electroporation is preferable, but the method is not limited thereto.

In the method for producing a founder animal of the present invention, the step of growing the first pluripotent stem cell (for example, a fertilized egg, an embryo, or the like) into a blastocyst can be carried out by any publicly-known method for growing a pluripotent stem cell into a blastocyst. The conditions for this are publicly known in the art, and described in Manipulating the Mouse Embryo, A LABORATORY MANUAL 3rd Edition (Cold Spring Harbor Laboratory Press, Cold Spring Harbor, New York)(incorporated herein by reference).

In the method for producing a founder animal of the present invention, the step of introducing the second pluripotent stem cell, which has an ability of complementing a deficiency due to the gene, into a blastocyst so as to produce a chimeric blastocyst may adopt any publicly-known method in the art as long as the second pluripotent stem cell can be introduced into a blastocyst. Examples of such a method include an injection method and an aggregationmethod; however, the method is not limited to these.

In the method for producing a founder animal of the present invention, a method for producing an individual from a chimeric blastocyst may adopt a publicly-known technique in the art. Generally, the chimeric blastocyst is returned to a surrogate parent, and then pseudo-pregnancy of the surrogate parent is caused so as to grow a resulting individual in the womb of the surrogate parent. However, the method is not limited to this technique.

In the method for producing a founder animal of the present invention, selecting of one having an organ or body part thereof complemented can be carried out by using any technique allowing confirmation of complementation of the organ or body part.

An example of such a technique is distinguishing an identifier derived from the second pluripotent stem cell. The "identifier" herein is any factor which allows specifying of a certain individual or species and identifying of the origin thereof, and is also referred to as "ID" in its abbreviation. Such an identifier could be, for example, a genomic sequence unique to the second pluripotent stem cell. Alternatively, regarding such selecting, by using the second pluripotent stem cell which is labeled or can be labeled (including one which can be a label by gene expression), selecting in the method for producing a founder mouse of the present invention may be carried out by identifying the label. In addition, it is understood that those in the art can carry out the selecting by modifying this technique as necessary.

### (Method of Organ Regeneration Using Founder Animal)

In another aspect, the present invention provides a method for producing any one of a target organ and body part. The method comprises the steps of: A) providing a founder animal, in which a deficiency responsible gene codes for a factor which causes a deficiency of the any one of a target organ and a target body part; B) obtaining an ovum from the animal, and then growing the ovum into an blastocyst; C) introducing a target pluripotent stem cell into the blastocyst so as to produce a chimeric blastocyst, the target pluripotent stem cell having a desired genome capable of complementing a deficiency caused by the deficiency responsible gene; and D) producing an individual from the chimeric blastocyst, and then obtaining the any one of a target organ and a body part from the individual.

Here, the step D) can be carried out by generating the chimeric blastocyst in a womb of the animal (ethically, preferably a non-human host mammal), obtaining a litter, and to obtain the target organ from an individual of the litter.

### (Formation of Pancreas)

The formation of a pancreas can be investigated by performing macroscopic or microscopic morphological analysis, gene expression analysis, and the like, using methods, such as visual inspection, microscopic observation after staining, and observation using fluorescence.

For example, by performing visual inspection, the actual presence or absence of the organ, and features of the organ, such as the external appearance, can be investigated. Together with such a macroscopic morphological analysis, a tissue obtained after general tissue staining, such as hematoxylin-eosin staining, may be observed microscopically using a microscopy. Such microscopic observation allows investigations to be performed, even on various concrete cellular compositions within the pancreas.

Furthermore, the gene expression analysis using fluorescence in such a way as to emit fluorescence according to conditions may also be performed. For example, a blastocyst derived from a mouse Pdx1-Lac-Z knock-in mouse, in which LacZ gene has been knocked in (also knocked out) into the Pdx1 gene locus, to be described in Examples herein, has the following characteristics. When a fluorescent-labeled ES cell is used in a wild type (+/+) or heterozygous (+/-) individual, mottled fluorescence in a chimeric state is shown even though the contribution of the ES cell is observed. On the other hand, in a homozygous (-/-) individual, uniform fluorescence is shown because the pancreas is constructed by a cell that is completely derived from ES cells. Using such characteristics, it is possible to conveniently examine which genotype a target organ or a cell constituting the target organ has with respect to the Pdx1 gene. iPS cells and mGS cells can be used. For example, in order to prepare an iPS cell, Okita K et al., Generation of germline-competent induced pluripotent stem cells. Nature 448 (7151) 313-7 (2007) or the like may be referred. In the case of an iPS cell line called Nanog-iPS, which was produced based on this document, since the iPS cell line is not marked, the cells cannot be distinguished from the embryos of the host when used in the production of chimera, and it cannot be discriminated whether the complementation of organ has been achieved. Therefore, in order to solve this problem, a fluorescent dye can be introduced into this Nanog-iPS cell line, thereby being capable of carrying out an experiment with the same protocol as the case of using the ES cell. If the cell such as described above is used, it is possible to produce an organ with the same protocol as the case of using the ES cell, and to clarify the origin.

The target organ obtained according to the present invention is characterized by being derived completely from the different individual mammal. In a conventional method, a chimera was regenerated. While not wishing to be bound by theory, it is conceivable that this is because the transcription factor is necessary to the functions of the deficient gene during the development process, particularly to the differentiation and maintenance of the stem/precursor cells of each organ during the process of the formation of the organ. In the present invention, ES cells, iPS cells, mGS cells, and the like can be used, and they may be labeled as described above.

The present invention also provides a mammal produced by the method of the present invention. It is considered that the animal itself is also valuable as an invention because such an animal having such a target organ could not be produced in the past. While not wishing to be bound by theory, it is conceivable that the reason why such an animal could not be produced in the past is because the defected organ due to the gene deficiency is necessary for survival, and there was no way to rescue them.

Furthermore, the present invention also provides use of a non-human host mammal having an abnormality associated with a lack of development of a target organ in the development stage, for generation of the target organ. Use of a host cell for such a use was not sufficiently assumed in the past. Accordingly, it is considered that the mammal itself is also valuable as an invention. While not wishing to be bound by theory, it is conceivable that the reason why such animals could not be produced in the past is because the deficient organ due to the gene deficiency was necessary for survival and it was impossible to maintain a target individual to sexual maturity.

### (Cases of Other Stem Cells)

As stem cells other than an ES cell, for example, an iPS cell, an mGS cell, and the like may be used. For example, in order to produce iPS cells, Okita K et al., ibid may be referred. Similarly for mGS cells, a publicly-known technique in the art (Mito Kanatsu-Shinohara et al., Generation of Pluripotent Stem Cells from Neonatal Mouse Testis. Cell. vol. 119, 1001-1012 (2004)) can be used. In the case of having no marking, the cells cannot be distinguished from the embryos of the host when used in the production of chimera, and it cannot be discriminated whether the complementation of organ has been achieved. Therefore, in order to solve this problem, a fluorescent dye can be introduced into this Nanog-iPS cell line, thereby being capable of carrying out an experiment with the same protocol as the case of using the ES cell. Then, if the cell such as described above is used, it is possible to produce an organ with the same protocol as the case of using the ES cell, and to clarify the origin.

### (iPS Cells)

iPS cells can be produced by other methods rather than Okita K et al., ibid. Specifically, iPS cells can be produced when somatic cells are brought into contact with a reprogramming factor (which may be a single factor or in combination of multiple factors) so as to induce initialization. Examples of such initialization and reprogramming factor include the following. For example, in Examples of the present invention, iPS cells were uniquely produced by the inventors using 3 factors (Klf4, Sox2, and Oct3/4, which are typical "reprogramming factors" used in the present invention), and a fibroblast collected from a tail of a GFP transgenic mouse. Other combinations than this, for example, 4 factors including Oct3/4, Sox2, Klf4, and c-Myc, which are called Yamanaka factors, may also be used, and a modified method thereof may also be used. It is also possible to establish iPS cells using n-Myc instead of c-Myc, and using a lentivirus vector, which is a type of retrovirus vector (Blelloch R et al., (2007). Cell Stem Cell 1: 245-247). Further, human iPS cells have been successfully established by introducing 4 genes, which are Oct3/4, Sox2, Nanog, and Lin28, into a fetal lung-derived fibroblast or neonatal foreskin-derived fibroblast (Yu J, et al. , (2007). Science 318: 1917-1920), and these may also be used in the present invention.

It is also possible to produce human iPS cells from a fibroblast-like synoviocyte and a neonatal foreskin-derived fibroblast by using mouse genes homologous to human genes, Oct3/4, Sox2, Klf4, and c-Myc, which were used in establishing mouse iPS cells (Takahashi K, et al., (2007). Cell 131: 861-872). It is also possible to establish human iPS cells by using 6 genes which are hTERT and SV40 large T in addition to the four genes including Oct3/4, Sox2, Klf4, and c-Myc (Park IH, et al., (2007). Nature 451: 141-146). Further, although at a low efficiency, establishment of iPS cells in mouse and human by only using 3 factors, Oct-4, Sox2, and Klf4, without introduction of the c-Myc gene has been indicated to be possible. Since the iPS cells are successfully prevented from turning into cancer cells, these can also be used in the present invention (Nakagawa M, et al., (2008). Nat Biotechnol 26: 101-106.; Wering M, et al., (2008). Cell Stem Cell 2: 10-12). In addition, iPS cells can also be produced by using not only a gene product but also low molecular weight compounds, and these can also be used in the present invention (refer to Shi et al., A combined chemical and genetic approach for the generation of induced pluripotent stem cells. Cell Stem Cell. 2008 Jun 5; 2 (6): 525-528).

### (Points to Remember when Using Various Animals)

The cases of using animals other than a mouse can be performed by applying a technique described in Examples herein upon paying attention to the following points. For example, regarding the production of a chimera in other species of animals, specifically in a species other than mice, there are more reports of chimeras into which an embryo or an inner cell mass, which is a part of an embryo and is an origin of an ES cell is injected, rather than of establishment of pluripotent stem cells having an ability to form a chimera (rat: (Mayer, J. R. Jr. & Fretz, H. I. The culture of preimplantation rat embryos and the production of allophenic rats. J. Reprod. Fertil. 39, 1-10 (1974)); cattle: (Brem, G. et al. Production of cattle chimerae through embryo microsurgery. Theriogenology. 23, 182 (1985)); pig: (Kashiwazaki N et al., Production of chimeric pigs by the blastocyst injection method, Vet. Rec. 130, 186-187 (1992)). However, even when a chimera into which an inner cell mass is injected is used, the method described herein may be applied. By using an inner cell mass as described above, it is substantially possible to complement a defected organ of a defected animal. In other words, for example, the above-described cells are each cultivated to grow into a blastocyst in vitro, a portion of inner cell mass is physically separated from thus obtained blastocyst, and then, the portion may be injected into a blastocyst. A chimeric embryo can be produced by aggregating the 8 cell-stage ones or morulas in mid-course.

In the case of using a chimera into which an inner cell mass has been injected instead of a pluripotent stem cell, such as an ES cell and an iPS cell, it should be noted in Examples described herein that the following points need to be modified or corrected in use. However, it is understood that these are techniques falling within the scope of a well-known technique in the art.

In the case of using a chimera into which an inner cell mass has been injected instead of a pluripotent stem cell, such as an ES cell and an iPS cell, since it is not a cell line as different from the ES cell and iPS cell, a process of producing an embryo separately (egg collection after natural crossbreeding, or artificial fertilization) is needed. Since such a protocol is disclosed in the above documents (rat: (Mayer, J. R. Jr. & Fretz, H. I. The culture of preimplantation rat embryos and the production of allophenic rats. J. Reprod. Fertil. 39, 1-10 (1974)); cattle: (Brem, G. et al. Production of cattle chimerae through embryo microsurgery. Theriogenology. 23, 182 (1985)); pig: (Kashiwazaki N et al., Production of chimeric pigs by the blastocyst injection method, Vet. Rec. 130, 186-187 (1992)), these documents, if necessary, are incorporated herein by reference.

### (General Techniques)

The molecular biological method, the biochemical method, and the microbiological method used in the present specification are well known and commonly used in the art, and are disclosed in, for example: Sambrook J. et al. (1989). Molecular Cloning: A Laboratory Manual, Cold Spring Harbor, and its 3rd Ed. (2001); Ausubel, F. M. (1987). Current Protocols in Molecular Biology, Greene Pub. Associates and Wiley-Interscience; Ausubel, F. M. (1989). Short Protocols in Molecular Biology: A Compendium of Methods from Current Protocols in Molecular Biology, Greene Pub. Associates and Wiley-Interscience; Innis, M. A. (1990). PCR Protocols: A Guide to Methods and Applications, Academic Press; Ausubel, F. M. (1992).Short Protocols in Molecular Biology: A Compendium of Methods from Current Protocols in Molecular Biology, Greene Pub. Associates; Ausubel, F. M. (1995).Short Protocols in Molecular Biology: A Compendium of Methods from Current Protocols in Molecular Biology, Greene Pub. Associates; Innis, M. A. et al. (1995) . PCR Strategies, Academic Press; Ausubel, F. M. (1999). Short Protocols in Molecular Biology: A Compendium of Methods from Current Protocols in Molecular Biology, Wiley, and annual updates; Sninsky, J. J. et al. (1999). PCR Applications: Protocols for Functional Genomics, Academic Press; separate-volume laboratory medicine "Experimental technique for gene transfer & expression analysis" Yodosha, 1997; and so on. The parts (or could be all) of these documents related to the present description are incorporated herein by reference.

A DNA synthesis technique and nucleic acid chemistry for producing an artificially synthesized gene are disclosed in, for example: Gait, M. J. (1985). Oligonucleotide Synthesis: A Practical Approach, IRLPress; Gait, M. J. (1990). Oligonucleotide Synthesis: A Practical Approach, IRL Press; Eckstein, F. (1991). Oligonucleotides and Analogues: A Practical Approac, IRL Press; Adams, R. L. et al. (1992). The Biochemistry of the Nucleic Acids, Chapman & Hall; Shabarova, Z. et al. (1994). Advanced Organic Chemistry of Nucleic Acids, Weinheim; Blackburn, G. M. et al. (1996). Nucleic Acids in Chemistry and Biology, Oxford University Press; Hermanson, G. T. (1996). Bioconjugate Techniques, Academic Press; and so on. The parts of these documents related to the present description are incorporated herein by reference.

Reference documents cited herein, such as science documents, patents, and patent applications, are incorporated herein by reference in their entirety to an extent that each of which is specifically described.

The preferred embodiments have been described for easy understanding of the present invention. Hereinafter, the present invention will be described based on examples; however, the above description and the following examples are provided only for exemplary purposes and are not provided for the purpose of limiting the present invention. Therefore, the scope of the present invention is not limited to the embodiments or examples which are specifically described herein, and is limited only by the claims.

### Examples

### (Example 1)

In the present example, a mouse was selected to be a founder animal, and pancreas was selected as an organ to be defected. Further, for preparation of a knockout mouse that was characterized by pancreas deficiency, a Pdx1 gene was used.

### (Mouse Used)

As a knockout mouse that was characterized by pancreas deficiency, Pdx1^{wt/LacZ} and Pdx1^{LacZ/LacZ} (founder) were used. A blastocyst derived from a mouse in which LacZ gene had been knocked in (also knocked out) at a Pdx1 gene locus (Pdx1-LacZ knock-in mouse) was used.

### (Pdx1-LacZ knock-in Mouse)

In regard to the production of a construct, it can be produced, specifically based on the published article in Development 122, 983-995 (1996). In brief, the procedure is as follows. As for the arm of the homologous region, a product cloned from a λ clone including the Pdx1 region can be used. In the present example, an arm donated by Professor Yoshiya Kawaguchi at the Laboratory of Surgical Oncology, Kyoto University Graduate School of Medicine, was used.

### (Technique of Transgenic Knock-In: Pdx1-LacZ Knock-In Mouse)

A clone obtained by introducing the construct described above into ES cells by electroporation, performing positive/negative selection, and then screening by Southern Blotting, was injected into a blastocyst so as to produce a chimeric mouse. Subsequently, a cell line developed into the germline is established, and the genetic background can be backcrossed into a C57BL/6 strain to produce the mouse.

### (Breeding)

Next, in the present example, heterozygous mice thus established were bred and used (Fig. 1). In regard to the knock-in mice described above, since the mice could not survive homozygosity (died in about one week after birth), heterozygous mice were bred, and the embryos were recovered.

### (Procedure for Maintenance of Mouse and Confirmation)

ES cells were injected into blastocysts under a microscope using a micromanipulator. In this instance, a line called G4.2, which was marked with EGFP, was used as the ES cell (donated by Professor Niwa Hitoshi at RIKEN CDB). A marked ES cell or the like which is equivalent to this line may also be used. The embryo after the injection was transplanted into the womb of a surrogate parent, and thus a litter was obtained.

For thus-obtained litter, if the litter is knock-in mice, the probability of the animals being homologous is 1/4. For this reason, it is necessary to decide which mouse has the desired "pancreas-deficient + ES cell-derived pancreas." Therefore, a hit mouse was determined by collecting the cells of blood and tissues from both animals, isolating cells that were found to be EGFP-negative (cells which were not derived from ES cells, but which were derived from the injected embryos) by a flow cytometer, extracting the genomic DNA, and detecting the genotype by a PCR method (Fig. 2). Primers used were as follows.
Forward (Fw) : TTC ATG CGA CGG TTT TGG AAC (Sequence number 1)
Reverse 1 (Rv1): TTC AAC ATC ACT GCC AGC TCC (Sequence number 2)
Reverse (Rv2): TGT GAG CGA GTA ACA ACC (Sequence number 3)

When produced by this method in which heterozygous indivisualswere bred, a resulting litter is expected to be wild type: heterozygous: KO=1: 2: 1 according to Mendelian inheritance. Accordingly, in order to specify a KO individual within the litter, genotyping should be carried out using host-derived cells in peripheral blood as described above to specify the genotype.

As the first step of confirming whether or not the complemented organ was functioning normally, analysis of expression of functional markers was carried out in the neonatal period in which morphological observation of pancreas is easy.

Fig. 3 shows images of frozen sections, which were prepared from pancreases of mice dissected in the neonatal period, and then subjected to immunostaining. The immunostaining was carried out using each of antibodies including: an anti-α-amylase antibody (purchased from SIGMA CORPORATION, cat. #A8273) as a marker of exocrine tissue; an anti-insulin antibody (purchased from NICHIREI Biosciences Inc., cat. #422421), an anti-glucagon antibody (purchased from NICHIREI Biosciences Inc., cat. #422271), and an anti-somatostatin antibody (purchased from NICHIREI Biosciences Inc., cat. #422651) as markers of endocrine tissue; and a DBA-Lectin (purchased from Vector Laboratories, cat. #RL-1032) as a marker of pancreatic duct. As being positive to all of these, it was indicated that the complemented pancreas functioned normally.

From this result, expression of almost all the functional markers was confirmed. Therefore, it was inferred that the complemented pancreas had normal functions which were enough for its survival.

Then, as the next step of confirming whether or not the complemented organ functioned normally, measurement of blood glucose level in adult mice was carried out (Fig. 4).

Fig. 4 shows result of pancreatic function evaluation using blood glucose level as an indicator carried out in mice with a pancreas complemented. (1) shows averages and standard deviations of steady state blood glucose levels, which were measured using Medisafe Mini GR-102 (purchased from TERUMO CORPORATION), of matured mice with a pancreas complemented. As controls, levels of chimeric mice having Pdx1 alleles in a heterozygous state and an STZ-DM model having a decreased pancreatic function are shown. In the meantime, (2) shows result of measurement of changes in the blood glucose level after a glucose tolerance test, the measurement being carried out using the above-described Medisafe Mini. These results show normality of the ability to regulate blood glucose level, and indicate that the thus produced mice with a pancreas complemented were able to survive over a long period of time even when used as a founder.

Specifically, as having a blood glucose level, which was once elevated but had gone back to the normal level after the glucose tolerance test similarly to that of the hetero chimera (+/-) used as a control, the produced KO chimeras (founders) did not show any symptoms of diabetes or the like. Thus, the possibility of long-term survival was indicated.

Next, it was attempted to reveal using a litter obtained from breeding with a hetero individual whether or not the chimera can transmit the phenotype to the next generation as a founder. Fig. 5 shows the process and result. Genomic DNA extracted from a tail of a thus obtained litter was subj ected to PCR using the primers used in the section (Procedure for Maintenance of Mouse and Confirmation). As a result, it was revealed that only hetero or knockout individuals could be obtained. This strongly suggested that the founder was a knockout individual and capable of transmitting the phenotype to the next generation.

Specifically, since the breeding was performed between knockout (KO) and hetero mice, an obtained litter was expected to be a KO or hetero individual theoretically at a probability of 1/2 according to Mendelian inheritance. Then, result as expected was demonstrated.

This allows obtaining of a KO individual in the next generation at a probability of 100% in breeding between KO individuals each with a pancreas, for example, complemented. Accordingly, it is expected that analysis using a KO individual will be able to be carried out much more easily.

### (Mouse Used)

As a transgenic mouse characterized by pancreas deficiency, a mouse (Pdx1-Hes1 mouse) produced by injecting a construct in which a Hes1 gene is connected downstream of the Pdx1 promoter region into a mouse egg in the pronuclear stage was used. In regard to the production of the construct, the construct can be produced by inserting a Hes1 gene (an mRNA whose NCBI Accession Number is NM_008235) at the region of the Pax6 gene in the construct including the Pdx1 promoter region, the construct having been used in the published article in Diabetologia 43, 332-339 (2000).

### (Technique of Transgenic)

The above construct was injected using a microinjector into an egg in the pronuclear stage obtained from breeding between a C57BL6 mouse and a BDF1 mouse (purchased from Japan SLC, Inc.), and a resulting egg was transplanted into a surrogate parent to produce a transgenic mouse. Fig. 6 shows an example of a mouse with a pancreas complemented, the mouse being produced by blastocyst complementation.

The degree of formation of pancreas differs depending on the expression level of Hes1 which is expressed under the Pdx1 promoter (expressed especially in a fetal pancreas). When the expression is high (that is, the copy number is high), a deficiency of pancreas is indicated. Regeneration of pancreas by blastocyst complementation in the Pdx1-Hes1 transgenic mouse has been shown. Using this mouse, it is possible to produce a pancreas derived from ES cells.

From this, it was demonstrated that the pancreas of a transgenic mouse thus produced could be complemented in the same manner as that of the Pdx1 knockout mouse.

### (Production of Founder Transgenic Mouse)

Since it was known that the above-described transgenic mouse would die after birth, a strategy shown in Fig. 7 was employed to produce a founder of such a mouse.

In a brief description, an embryo into which the Pdx1-Hes1 transgene had been injected was cultured to become a blastocyst, and an ES cell or the like was injected into a thus obtained blastocyst using a micromanipulator under a microscope so as to complement a deficiency of pancreas. In this instance, an ES cell line called G4.2, which was marked with EGFP (donated by Professor Niwa Hitoshi at RIKEN CDB) was used as the ES cells as well as in the section of knockout. Alternatively, a marked ES cell or the like which is equivalent to this ES cell line may be used. The embryo after the injection was transplanted into the womb of a surrogate parent, and thus a litter was obtained. When double embryo manipulations are applied in which a transgene is introduced into an embryo and then an ES cell is injected into the embryo, it is possible to complement a pancreas in the first-generation transgenics. Accordingly, these may be a founder animal which is capable of transmitting the phenotype of pancreas deficiency.

In a conventional method of producing a transgenic mouse, a gene is introduced into an egg cell in the pronuclear stage, and then a resulting egg cell is transplanted. By doing so, however, a mouse with a phenotype of pancreas deficiency ends up dying after birth. Therefore, the present invention provides a technique which could not be achieved by the conventional method.

Fig. 8 shows result in a course of the production summarized in a table.

In the course of the production, chimeras were determined by their coat colors. Since the donor ES cells were derived from the mouse strain 129 (agouti) and the host embryo was derived from C57BL6xBDF1 (black), it was possible to determine whether or not an individual was a chimera when its coat had fully grown. Determination of the transgenic was carried out by detecting the transgene by PCR on genomic DNA extracted from a tail thereof.

If the litter is transgenic, the probability of the transgene being transmitted to the next generation is 1/2. For this reason, it is necessary to decide which mouse has the desired "pancreas-deficient + ES cell-derived pancreas." Therefore, as shown in Fig. 9, individuals of the litter were each bred with a wild type mouse. Then, transmission of the transgene was confirmed by detecting a genotype by PCR on genomic DNA extracted from tails of a resulting litter, and morphology of pancreases of the litter was observed. The following primer set was used for the PCR.
Froward (Fw) : TGA CTT TCT GTG CTC AGA GG (Sequence Number 4)
Reverse (Rv) : CAA TGA TGG CTC CAG GGT AA (Sequence Number 5)

The forward primer used was prepared so as to hybridize with a nucleotide sequence corresponding to the Pdx1 promoter region, while the reverse primer was prepared so as to hybridize with a nucleotide sequence of Hes1 cDNA (an mRNA whose Accession Number is NM_008235). Since such a Pdx promoter and Hes1 cDNA existing in the neighborhood cannot occur in wild type mice, it is possible to detect the transgene efficiently by PCR using these primers.

From the experiment above, result was obtained which suggested that #37 in Fig. 8 was a founder mouse capable of causing pancreas deficiency in the next generation.

It is expected that application of such a method not only to mice but other large-size animals and the like will allow more efficient production of transgenic animals and knockout animals having a lethal phenotype.

Fig. 9 shows a process and result of the present example.

A thus produced transgenic and chimeric individual was bred with a wild type. It was to be revealed whether or not the phenotype of pancreas deficiency was transmitted to the next generation by carrying out morphological analysis of pancreas of a litter or PCR on the genomic DNA. If a transgenic-chimera can be a founder, a mouse with pancreas deficiency should be born in the next generation. Among the founder candidates shown in Fig. 8, #37 was successful in causing deficiency of pancreas in the next generation. According to this, it was suggested that #37 showed normality after birth as its pancreas was complemented during the production. This indicated that, even when a transgenic is used as described above, it is made possible to produce a founder allowing efficient production of mice, such as an organ deficient mouse, which would die in the fetal stage and after birth.

From the above, it was demonstrated that organ deficient animals, even mice including ones with pancreatic agenesis due to forced expression of HES-1 and even Pdx-1 knockout mice, can be rescued from death by blastocyst complementation and used as founders.

### (Example 2: Example of Using iPS Cell)

In the present example, using iPS cells instead of ES cells, an experiment similar to that in Example 1 was carried out. It should be noted that iPS cell is basically a pluripotent stem cell line and has properties very similar to those of ES cell. Therefore, the protocol performed in Example 1 can be carried out without any modification. Specifically, there is no difference in the method of culture and the method of introduction into an embryo; thus, it is understood that the present example can be carried out by using those desecribed in Example 1.

### (Example of Preparation of iPS Cell)

The inventors produced iPS cells with 3 factors (Klf4, Sox2, and Oct3/4) by using a fibroblast collected from a tail of a GFP transgenic mouse. The protocol used for the production is as follows. The scheme is shown in Fig. 10, and shown in detail in Fig. 11a.

### (Establishment of GFP Mouse Tail-Tip Fibroblast (TTF))

Approximately 1 cm of a tail of a GFP transgenic mouse was collected, peeled, and minced into 2 to 3 pieces. Then, these pieces were placed on MF-start medium (TOYOBO, Japan), and cultured for 5 days. Fibroblasts which appeared there were transferred to a fresh culture dish, and subcultured for several passages to be used as tail tip fibroblasts (TTF).

### (Introduction of +3 Factors (Reprogramming Factors))

A supernatant from a virus producing cell line (293gp or 293GPG cell line) produced by introducing a target gene and a virus envelop protein was collected, concentrated by centrifugation, and then frozen for preservation to be used as a virus fluid. The virus fluid was added to a culture medium of TTF cells which had been subcultured on a previous day to achieve 1×10⁵ cells/6-well plate. This completed the introduction of the 3 factors (reprogramming factors).

### (Culture in ES-cell Medium For 25 to 30 days)

On the next day after the introduction of the 3 factors (reprogramming factors), the culture medium was replaced with a culture medium for ES cell culture, and the culture was continued for 25 to 30 days. During this, culture medium was replaced every day.

### (Pick-up of iPS Colonies and Establishment of iPS Cell Line)

iPS cell-like colonies appeared after the culture were picked up using a yellow tip (for example, available from Watson), dissociated into single cells in 0.25% trypsin/EDTA (Invitrogen Corp.), and spread on a freshly prepared mouse embryonic fibroblasts (MEF).

### (Result)

It was proved that the iPS cell line established by the above-described method has properties of iPS cells shown in Figs. 11b to f, that is, being undifferentiated and having totipotency.

Fig. 11 shows result of the above-described experiment. As shown in Fig. 11b, morphology of two of thus established iPS cell lines was photographed by a microscope equipped with a camera. The conditions were as follows.

After subculturing the iPS cells after the pick-up, they were observed and photographed when they reached the semi-confluent stage.

It was found that morphologically ES cell-like undifferentiated colonies were formed.

As shown in Fig. 11c, the iPS cells were photographed under a fluorescent microscope, and subjected to staining using an alkaline phosphatase staining kit (Vector Laboratories, Inc., Cat. No. SK-5200). The conditions were as follows.

After a bright-field image and a GFP fluorescence image were observed and photographed by a microscope equipped with a camera, the culture medium was removed from the iPS cell culture dish, and the dish was washed with a phosphate buffer saline (PBS). Then, a fixing solution containing 10% formalin and 90% methanol was added to the dish, thereby performing a fixing treatment for 1 to 2 minutes. After washing the resultant dish once with a washing solution (0.1M Tris-HCl (pH9.5)), a staining solution included in the above kit was added to the dish, and the dish was left to stand in dark for 15 minutes. Thereafter, the dish was again washed with the washing solution, and then observed and photographed.

As shown in Fig. 11c, it was found that, as having been derived from a GFP mouse, the iPS cells produced in the present example constantly expressed GFP, and showed a high-level of alkaline phosphatase activity that is characteristic of undifferentiated cells.

As shown in Fig. 11d, for the purpose of identifying the 3 factors inserted into the genomic DNA in the establishment of the iPS cells, the genomic DNA was extracted from the iPS cells and subjected to PCR. The conditions were as described below.

The genomic DNA was extracted from 1x10⁶ cells using DNA mini kit (Quiagen Co., Ltd.) according to the manufacturer's protocol. Using thus extracted DNA as a template, PCR was carried out using the primers below.
Oct3/4
Fw (mOct3/4-S1120): CCC TGG GGA TGC TGT GAG CCA AGG (Sequence Number 6)
Rv (pMX/L3205) : CCC TTT TTC TGG AGA CTA AAT AAA (Sequence Number 7)
Klf4
Fw (Klf4-S1236) : GCG AAC TCA CAC AGG CGA GAA ACC (Sequence Number 8)
Rv (pMXs-AS3200) : TTA TCG TCG ACC ACT GTG CTG CTG (Sequence Number 9)
Sox2
Fw (Sox2-S768): GGT TAC CTC TTC CTC CCA CTC CAG (Sequence Number 10)
Rv (pMX-AS3200): same as above (Sequence Number 9)
c-Myc
FINS (c-Myc-S1093) : CAG AGG AGG AAC GAG CTG AAG CGC (Sequence Number 11)
Rv (pMX-AS3200): same as above (Sequence Number 9)

As a result, the insertion of the 3 factors was confirmed as shown in Fig. 11d.

As shown in Fig. 11e, a gene expression pattern unique to ES cell and expression of introduced genes were confirmed by reverse-transcription polymerase chain reaction (RT-PCR). The conditions were as described below.

1x10⁵ GFP-positive cells were sorted into Trizol-LS Reagent (Invitrogen Corp.) using a flow cytometer, mRNA was extracted from the cells, and cDNA was synthesized from the mRNA using ThermoScript RT-PCR System kit (Invitrogen Corp.) according to the attached protocol. Thus synthesized cDNA was used as a template to perform PCR. In regard to primers used, the same primers as those used in Fig. 11d above were used for transgene expression (notated as Tg in the drawing), while primers synthesized based on the report by Takahashi K & Yamanaka S (Cell 2006 Aug 25; 126 (4): 652-5.) or the like were used for other gene expression.

As shown in Fig. 11e, all the lines showed expression patterns approximately same as that of ES cell. Further, it was found that expression of the introduced gene (Tg) was inhibited by a high level of gene silencing activity of iPS cell.

As shown in Fig. 11f, thus established iPS cells were injected into a blastocyst to produce a chimeric mouse. The conditions were as follows.

Using an ovum collected from a BDF1 strain mouse (female, 8 week old) having been subjected to an ovarian superstimulation treatment by administration of PMSG and hCG hormones and a C57BL/6-derived sperm, in vitro fertilization (IVF) was performed to obtain a fertilized egg. The fertilized egg thus obtained was cultured to the 8 cell-stage/ morula, then frozen for preservation, and recovered the day before blastocyst injection. In regard to the iPS cells, those reached the semi-confluent stage were detached using 0.25% Trypsin/EDTA, and suspended in an ES-cell culture medium to be used for injection. Blastocyst injection was performed, in the same manner as the technique used for the blastocyst complementation, under a microscope using a micromanipulator. Going through culture after the injection, transplantation into the womb of an ICR strain surrogate mother was performed. In analysis, observation and photographing were carried out under a fluorescence stereomicroscope on embryonic day 13 and postnatal day 1.

As shown in Fig. 11f, iPS cell-derived cells (GFP positive) were confirmed in a fetal period and a neonatal period. Accordingly, it was suggested that the established iPS cell line possessed high multipotency.

### (Mouse Used)

As a knockout mouse that was characterized by pancreas deficiency, Pdx1^{wt/LacZ} and Pdxl^{LacZ/LacZ} (founders) were used. A blastocyst derived from a mouse in which LacZ gene had been knocked in (also knocked out) at a Pdx1 gene locus (Pdx1-LacZ knock-in mouse) was used.

### (Pdx1-LacZ knock-in Mouse)

A Pdx1-LacZ knock-in mouse was produced according to the protocol described in Example 1, except for using iPS cells instead of ES cells.

### (Founder Mouse)

### (Mouse Used)

As a transgenic mouse characterized by pancreas deficiency, a mouse (Pdx1-Hes1 mouse) produced by injecting a construct (Pdx1-Hes1 construct) in which a Hes1 gene is connected downstream of the Pdx1 promoter region into a mouse egg in the pronuclear stage was used (in regard to the production of the Pdx1-Hes1 transgenic mouse and its construct, refer to Example 1).

Transgenic mice produced as described above had different degrees of formation of pancreas depending on the expression level of Hes1 which is expressed under the Pdx1 (expressed especially in a fetal pancreas) promoter. When the expression is high (that is, the copy number is high), a deficiency of pancreas is indicated. Regeneration of pancreas by blastocyst complementation in the Pdx1-Hes1 transgenic mouse has been shown. Using this mouse, it is possible to produce a pancreas derived from iPS cells.

From this, it was demonstrated that a pancreas of the thus produced transgenic mouse could also be complemented in the same manner as that of the Pdx knockout mouse.

### (Production of Founder Transgenic Mouse)

Since it was known that the above-described transgenic mouse dies after birth, a founder of such a mouse was produced.

In a brief description, an embryo into which the Pdx1-Hes1 transgene was injected was cultured to become a blastocyst, and an iPS cell was injected into a thus obtained blastocyst using a micromanipulator under a microscope, thereby complementing a deficiency of pancreas. In this instance, an iPS cell marked with GFP, which was prepared as described above, was used as the iPS cell. Alternatively, a marked iPS cell or the like which is equivalent to this may be used. The embryo after the injection was transplanted into the womb of a surrogate parent, and thus a litter was obtained. When double embryo manipulations are applied in which a transgene is introduced into an embryo and then an ES cell is injected into the embryo, it is possible to complement a pancreas in the first-generation transgenics. Accordingly, these may be a founder animal which is capable of transmitting the phenotype of pancreas deficiency.

### (Breeding)

Next, in the present example,heterozygous mice thus established were bred and used. In regard to the knock-in mice described above, since the mice could not survive homozygosity (died in approximately one week after birth), Pdx1^{wt/LacZ} and Pdx1^{LacZ/LacZ} (founders) were bred, and resulting embryos were recovered.

### (Procedure for Maintenance of Mouse and Confirmation)

The above-described iPS cells were injected into a blastocyst under a microscope using a micromanipulator (Fig. 10, Blastocyst injection with iPS cells). In a conventional method, it was necessary to mark the iPS cells with GFP. This time, however, since iPS cells were established from somatic cells of a GFP mouse in advance, they did not need to be marked, and used as they were. It is needless to say that other marked iPS cells or the like which are equivalent to this strain may also be used. The embryos after the injection were transplanted into the womb of a surrogate parent, and thus a litter was obtained.

For thus obtained litter, if the litter is knock-in mice, the probability of the animals being homologous is 1/4. For this reason, it is necessary to decide on which mouse is the desired "pancreas-deficient + iPS cell-derived pancreas." Therefore, a hit mouse was determined by collecting the cells of blood and tissues from both animals, isolating cells that were found to be GFP-negative (cells which were not derived from iPS cells, but derived from the injected embryos) by a flow cytometer, extracting the genomic DNA, and detecting the genotype by a PCR method. The PCR primers used were as follows.
Forward (Fw) : TTC ATG CGA CGG TTT TGG AAC (Sequence Number 1)
Reverse 1 (Rv1): TTC AAC ATC ACT GCC AGC TCC (Sequence Number 2)
Reverse (Rv2): TGT GAG CGA GTA ACA ACC (Sequence Number 3)

When produced by this method in which heterozygous individualswere bred, a resulting litter is expected to be wild type: heterozygous: KO =1: 2: 1 according to Mendelian inheritance. Accordingly, in order to specify a KO individual within the litter, genotyping should be carried out using host-derived cells in peripheral blood as described above to specify the genotype.

It was confirmed that the regenerated organ (pancreas) derived from thus produced founder mouse was functional, by using an organ derived from a pdx-1-/- mouse pup born from breeding between the founder mouse (male) and the pdx-1+/- heterozygous type (female) as described above. Specifically, as the first step to confirm whether or not a complemented organ functions normally, expression analysis of functional markers was carried out in a neonatal period in which morphological observation of pancreas can be easily performed. The result is shown in Fig. 11A and Fig. 11B. Fig. 11A shows pancreases exposed by dissection of neonates at 5 days after birth under a microscope and then photographed under a fluorescent microscope.

Fig. 11B shows images of frozen sections which were prepared from pancreases of mice dissected in the neonatal period and then subjected to immunostaining. Images of those which were stained using an anti-insulin antibody (purchased from NICHIREI Biosciences Inc., cat. #422421) as a marker of endocrine tissue as an antibody are shown. In addition to this, staining may be carried out using each of: an anti-α-amylase antibody (purchased from SIGMA CORPORATION, cat. #A8273), an anti-glucagon antibody (purchased from NICHIREI Biosciences Inc. , cat. #422271), and an anti-somatostatin antibody (purchased from NICHIREI Biosciences Inc., cat. #422651) as markers of exocrine tissue; and a DBA-Lectin (purchased from Vector Laboratories, cat. #RL-1032) as a marker of pancreatic duct. As being positive to insulin, it was understood without carrying out staining with other antibodies that the complemented pancreas functioned normally.

From this result, expression of almost all the functional markers was confirmed. Therefore, it was inferred that the complemented pancreas had normal functions which were enough for its survival.

Then, as the next step to confirm whether or not the complemented organ functioned normally, measurement of blood glucose level was carried out in adult mice.

Result of pancreatic function evaluation using blood glucose level as an indicator carried out in mice with a pancreas complemented can be taken into consideration. Averages and standard deviations of steady state blood glucose levels, which were measured using Medisafe Mini GR-102 (purchased from TERUMO CORPORATION), of matured mice with a pancreas complemented may be taken into consideration. As controls, levels of chimeric mice having Pdx1 alleles in a heterozygous state and an STZ-DM model having a decreased pancreatic function may be used. Further, result of measurement of changes in the blood glucose level after a glucose tolerance test may be taken into consideration, the measurement being carried out using the above-described Medisafe Mini. These results show normality of the ability to regulate blood glucose level, and indicate that the thus produced mice with a pancreas complemented were able to survive over a long period of time even when used as a founder.

Specifically, as having a blood glucose level, which was once elevated but had gone back to the normal level after the glucose tolerance test similarly to that of the hetero chimera (+/-) used as a control, the produced KO chimeras (founders) did not show any symptoms of diabetes or the like. Thus, the possibility of long-term survival can be indicated.

Next, it was attempted to reveal using a litter obtained from breeding with a hetero individual whether or not the chimera can transmit the phenotype to the next generation as a founder. Fig. 12 shows the result. Genomic DNA extracted from a tail of a thus obtained litter was subjected to PCR using the primers used in the section (Procedure for Maintenance of Mouse and Confirmation). As a result, it was revealed that only hetero or knockout individuals could be obtained. This strongly suggested that the founder was a knockout individual and capable of transmitting the phenotype to the next generation.

Specifically, since the breeding was performed between knockout (KO) and hetero mice, an obtained litter was expected to be a KO or hetero individual theoretically at a probability of 1/2 according to Mendelian inheritance. Then, result as expected was demonstrated.

This allows obtaining of a KO individual at a probability of 100% in breeding between KO individuals each with a pancreas, for example, complemented. Accordingly, it is expected that analysis using a KO individual will be able to be carried out much more easily.

### (Confirmation of Chimera)

Chimeras can be determined by their coat colors. Since the donor iPS cells were derived from the GFP transgenic mouse and the host embryo was derived from C57BL6xBDF1 (black), which is a wild type, it was possible to determine according to GFP fluorescence. Determination of transgenic was carried out by detecting the transgene by PCR on genomic DNA extracted from a tail thereof.

If the litter is transgenic, the probability of the transgene being transmitted to the next generation is 1/2. For this reason, it is necessary to decide which mouse has the desired "pancreas-deficient + iPS cell-derived pancreas." Therefore, individuals of the litter were each bred with a wild type mouse. Then, transmission of the transgene was confirmed by detecting a genotype by PCR on genomic DNA extracted from tails of a resulting litter, and morphology of pancreases of the litter was observed. The following primer set was used for the PCR.
Forward (Fw) : TGA CTT TCT GTG CTC AGA GG (Sequence Number 4)
Reverse (Rv) : CAA TGA TGG CTC CAG GGT AA (Sequence Number 5)

The forward primer used was prepared so as to hybridize with a nucleotide sequence corresponding to the Pdx1 promoter region, while the reverse primer was prepared so as to hybridize with a nucleotide sequence of Hes1 cDNA (an mRNA whose Accession Number is NM_008235). Since such a Pdx promoter and Hes1 cDNA existing in the neighborhood cannot occur in wild type mice, it is possible to detect a transgene efficiently by PCR using these primers. From the experiment above, result was obtained which suggested that thus obtained litter individual was a founder mouse capable of causing pancreas deficiency in the next generation.

It is expected that application of such a method not only to mice but other large-size animals and the like will allow more efficient production of transgenic animals and knockout animals having a lethal phenotype.

The thus produced transgenic and chimeric individual was bred with a wild type. It was to be revealed whether or not the phenotype of pancreas deficiency was transmitted to the next generation by carrying out morphological analysis of pancreases of a litter or PCR on genomic DNA. If a transgenic-chimera can be a founder, a mouse with pancreas deficiency should be born in the next generation. One successful in causing deficiency of pancreas in the next generation can be selected. It is suggested that such a mouse showed normality after birth as its pancreas was complemented during the production. Even when a transgenic is used as described above, it is made possible to achieve organ regeneration with iPS cells by using a founder allowing efficient production of mice, such as an organ deficient mouse, which would die in the fetal stage and immediately after birth.

From the above, it was demonstrated that organ deficient animals, even mice including ones with pancreatic agenesis due to forced expression of HES-1, and even Pdx-1 knockout mice, can be rescued from death using iPS cells by blastocyst complementation and used as founders.

### (Example 3: Example of Using mGS Cells)

In the present example, using mGS cells instead of ES cells or iPS cells, an experiment similar to that in Example 1 or 2 was carried out (refer to Mito Kanatsu-Shinohara et al., Generation of Pluripotent Stem Cells from Neonatal Mouse Testis. Cell. vol. 119, 1001-1012 (2004)). It should be noted that mGS cell is basically a pluripotent stem cell line and has properties very similar to those of ES cell or iPS cell. Therefore, the protocol performed in Example 1 or 2 can be carried out without or with modification as necessary. Specifically, there is no difference in the method of culture and the method of introduction into an embryo; thus, it is understood that the present example can be carried out by using those desecribed in Example 1 or 2 with modification as necessary.

### (Example 4: Example of Using Fertilized Egg Cells)

In the present example, using fertilized egg cells instead of ES cells or iPS cells, an experiment similar to that in Example 1 or 2 was carried out (refer to Mintz Experimental study of the developing mammalian egg: removal of the zona pellucida. Science. 138, 594-595 (1962)).

When fertilized egg cells are used, a method can be used in which fertilized egg cells are recovered from collection of eggs after artificial insemination or natural breeding depending on a target genetically modified embryo, the zona pellucida of the embryos are exposed to Acid Tyrode solution so as to be removed, resulting embryos are cultured at a high density in a single Petri dish so as to beaggregated, and thus a chimeric blastocyst is produced.

A method may be as well used in which a part

(blastomere) of a similarly treated fertilized egg cell or an inner cell mass of an embryo developed to blastocyst is mechanically detached and injected into a genetically modified blastocyst.

### (Example 5: Production of Founder Animal in Case of Kidney)

Next, in the present example, it was proved that a founder animal could be produced as well in the case of kidney deficiency.

In the present example, a mouse was selected as a founder animal, and, further, a Sa111 gene was used for production of a knockout mouse characterized by kidney deficiency.

### (Mouse Used)

As the knockout mouse characterized by kidney deficiency, a Sall1 knockout mouse (donated by Professor Ryuichi Nishinakamura at Institute of Molecular Embryology and Genetics, Kumamoto University) was used. Sa111 gene is a gene of 3969 bp, encoding a protein having 1323 amino acid residues, and this gene is a mouse homolog of the anterior-posterior region-specific homeotic gene spalt (sal) of Drosophila, and has been suggested by a pronephric tubule induction test in African clawed frogs to be important in kidney development (Nishinakamura, R. et al., Development, Vol. 128, p. 3105-3115, 2001, Asashima Lab, Tokyo University) . It was reported that this Sa111 gene was expressed and localized in the kidney, as well as in the central nervous system, auditory vesicles, heart, limb buds and anus in the mouse (Nishinakamura, R. et. al., Development, Vol. 128, p. 3105-3115, 2001).

The knockout mouse of this Sall1 gene (backcrossed to C57BL/6 strain and analyzed) has exon 2 and its subsequent parts in the Sall1 gene deleted, and thereby lacking all of the 10 zinc finger domains present in the molecule. It is conceivable that, as a result of the deletion, invasionof ureteric bud into the metanephric mesenchyme does not occur, thereby causing abnormality in the initial stage of kidney formation.

### (Production of Founder Transgenic Mouse)

Since it was known that the above-described transgenic mouse dies after birth, a founder of such a mouse was produced.

In a brief description, an embryo into which the Sall1 knockout transgene was injected was cultured to become a blastocyst, and an iPS cell was injected into a thus obtained blastocyst using a micromanipulator under a microscope, thereby complementing a deficiency of kidney. In this instance, one marked with GFP was used which was prepared as described above as the iPS cell. Alternatively, a marked iPS cell or the like which is equivalent to this may be used. The embryo after the injection was transplanted into the womb of a surrogate parent, and thus a litter was obtained. When double embryo manipulations are applied in which a transgene is introduced into an embryo and then an ES cell is injected into the embryo, it is possible to complement a kidney in the first-generation transgenics. Accordingly, these may be a founder animal which is capable of transmitting the phenotype of kidney deficiency.

### (Breeding)

Next, male and female heterozygote individuals (Sall1 (+/-)) of the Sall1 gene knockout mouse were bred, and thus the blastocyst stage fertilized eggs were collected by a uterine reflux method.

### (Procedure for Maintenance of Mouse and Confirmation)

iPS cells marked with GFP were injected into the collected blastocyst stage fertilized eggs under a microscope using a micromanipulator, and the embryos after the injection were transplanted into the womb of a surrogate parent, and thus a litter was obtained.

For thus obtained litter, if the litter is knock-in mice, the probability of the animals being homologous is 1/4. For this reason, it is necessary to decide which mouse has the desired"kidney-deficient + iPS cell-derived pancreas." Therefore, bone marrow cell were collected from the litter, isolating hematopoietic/precursor cells (c-Kit+, Sca-1+, Linage marker-: KSL cell) that were found to be GFP- by a flow cytometer, thus isolated cells were dropped onto a 96-well plate one by one. The cells were cultured under the condition of cytokine addition for 12 days to allow formation of colonies. Genomic DNA was extracted from these colonies, and the genotype was detected from a single cell by PCR so as to determine a hit mouse. Primers used were as follows.
Forward primer for identification of one derived from injected embryo (that is, a host):
For detection of mutant: AAG GGA CTG GCT GCT ATT GG (Sequence Number 12)
For detection of wild type: GTA CAC GTT TCT CCT CAG GAC (Sequence Number 13)
Reverse primer for identification of one derived from injected embryo (that is, a host):
For detection of mutant: ATA TCA CGG GAT GCC AAC GC (Sequence Number 14)
For detection of wild type: TCT CCA GTG TGA GTT CTC TCG (Sequence Number 15)

When produced by this method in which heterozygous indivisuals were bred, a resulting litter is expected to be wild type: heterozygous: KO=1: 2: 1 according to Mendelian inheritance. Accordingly, in order to specify a KO individual within the litter, genotyping using bone marrow cells as described above was carried out, and the genotype was specified (Fig. 13). It was found that #3 mouse was a Sall1 homo KO mouse.

In order to confirm whether or not the complemented organ functioned normally, morphological observation of kidney was performed. The neonatal chimeric individuals, which could be confirmed to be homozygotes (Sall1 (-/-)) by the above-described genotyping, had kidneys present in the retroperitoneal area. When these formed kidneys were observed under a fluorescent stereomicroscope, it was found that the kidneys were strongly GFP-positive (Fig. 13). This indicates that, in the homogyzote (Sall1 (-/-)), the kidneys were derived only from the mouse iPS cells transplanted into the inner space of the blastocyst stage fertilized eggs. On the other hand, in the heterozygote (sall1 (+/-)) individuals, since the kidneys were constituted of a chimera of the cells, which were derived from the heterozygote (Sall1 (+/-)) individuals, and the cells, which were derived from the transplanted iPS cells, confirmation was performed by obtaining cellular images that were positive for both the fluorescence of GFP and the immunohistochemically derived fluorescence using an anti-GFP antibody.

In the histological analysis of the kidneys obtained as a result of transplanting iPS cells into the homozygote (Sall1 (-/-)) blastocyst stage fertilized egg, mature functional glomeruli, which contained erythrocytes, in the loop cavity and mature renal tubular structures could be observed, and those mature cells could be confirmed to be mostly GFP-positive by an immunohistochemical analysis using an anti-GFP antibody.

From these results, it can be confirmed that, in the chimeric Sa111 knockout mouse (Sall1 (-/-)) created by the method described above, the kidneys formed in the individuals of a litter were formed from the iPS cells that had been transplanted into the inner space of the blastocyst stage fertilized eggs of the Sall1 knockout mouse (Sall1 (-/-)).

When KO individuals each with a kidney complemented are bred, it is possible to obtain a KO individual in the next generation at a probability of 100%. Accordingly, it is expected that analysis using a KO individual will be able to be carried out much more easily.

### (Example 6: Example of Using Animals Other Than Mouse)

In the present example, it is demonstrated that founder animals can be produced and organs can be produced even in the case of using animals other than mice. In regard to species other than mice, there are more reports of chimera into which an inner cell mass being an origin of an embryo or, especially among embryo, an ES cell is injected, rather than of establishment of pluripotent stem cells having an ability to form a chimera. Thus, organ generation can be carried out using this information. In the case of rat, a similar experiment can be carried out using the information described in Mayer, J. R. Jr. & Fretz, H. I. The culture of preimplantation rat embryos and the production of allophenic rats. J. Reprod. Fertil. 39, 1-10 (1974). In the case of cattle, a similar experiment can be carried out using the information described in Brem, G. et al. Production of cattle chimerae through embryo microsurgery. Theriogenology. 23, 182 (1985). In the case of pig, a similar experiment can be carried out using the information described in Kashiwazaki N et al., Production of chimeric pigs by the blastocyst injection method, Vet. Rec. 130, 186-187 (1992).

For example, in the present example, it is conceived that similar experiments can be carried out on animal species (rat (transgenic), pig (transgenic, knockout), and cattle (transgenic, knockout)) which are considered to be applicable to production of genetically modified animals, as examples rather than mouse. In these species, utilization of the fertilized egg cells described in Example 4 is assumed.

By this, it is possible to produce founder rat, pig, cattle, and the like, which are modified to have a lethal gene.

As described above, in the cases of using a rat, a pig, and cattle, similar experiments can be carried out as well in accordance with Examples 1 to 3.

As discussed above, the present invention was illustrated using preferred embodiments of the present invention, but it is understood that the scope of the present invention should be construed only by the claims. It is understood that the patents, patent applications, and articles cited herein should be such that the disclosures thereof should be incorporated into the present description by reference, as with the disclosures themselves are specifically described in the present description.

## Claims

1. An animal, which includes a deficiency responsible gene coding for a factor which causes a deficiency of any one of an organ and a body part and gives any one of no possibility of survival and difficulty in survival if the factor functions, and in which the any one of an organ and a body part is complemented by blastocyst complementation.

2. The animal according to claim 1, wherein the any one of an organ and a body part to be complemented is labeled.

3. A method for producing the animal according to claim 1, the method comprising the steps of:
A) providing a first pluripotent stem cell having the deficiency responsible gene;
B) growing the first pluripotent stem cell into a blastocyst;
C) introducing a second pluripotent stem cell into the blastocyst so as to produce a chimeric blastocyst, the second pluripotent stem cell having an ability to complement a deficiency caused by the deficiency responsible gene;
D) producing an individual from the chimeric blastocyst, and then selecting an individual in which the any one of an organ and a body part has been complemented by the second pluripotent stem cell.

4. The method according to claim 3, wherein the first pluripotent stem cell is any one of an inner cell mass (ICM), a fertilized egg, and an embryo.

5. The method according to claim 3, wherein
the gene is a foreign gene, and
the step A) includes introducing the foreign gene into the first pluripotent stem cell.

6. The method according to claim 3, wherein the second pluripotent stem cell is any one of an egg cell, an embryonic stem cell (ES cell), and an induced pluripotent stem cell (iPS cell).

7. The method according to claim 3, wherein the step D) includes returning the chimeric blastocyst into a surrogate parent, causing pseudo-pregnancy thereof, and thereby producing the individual.

8. The method according to claim 3, wherein the selecting is achieved by distinguishing an identifier derived from the second pluripotent stem cell.

9. The method according to claim 3, wherein
the second pluripotent stem cell is labeled, and
the selecting includes identifying the label.

10. A method for producing any one of a target organ and a target body part, the method comprising the steps of:
A) providing the animal according to claim 1, in which the deficiency responsible gene codes for a factor which causes a deficiency of the any one of a target organ and a target body part;
B) obtaining an ovum from the animal, and then growing the ovum into a blastocyst;
C) introducing a target pluripotent stem cell into the blastocyst so as to produce a chimeric blastocyst, the target pluripotent stem cell having a desired genome capable of complementing a deficiency caused by the deficiency responsible gene; and
D) producing an individual from the chimeric blastocyst, and then obtaining the any one of a target organ and a body part from the individual.

11. The method according to claim 10, wherein the step D) includes developing the chimeric blastocyst in a womb of the animal to obtain a litter, and obtaining the target organ from an individual of the litter.

12. The method according to claim 10, wherein the target pluripotent stem cell is any one of an ES cell and an iPS cell.

13. The method according to claim 10, wherein the target pluripotent stem cell is derived from a mouse.

14. The method according to claim 10, wherein the any one of a target organ and a target body part is any one of a pancreas and a kidney.

15. The method according to claim 10, wherein the animal is a mouse.

16. The method according to claim 15, wherein the mouse is any one of a Pdx-1 knockout mouse, a Pdx1-Hes1 transgenic mouse, and a Sall1 knockout mouse.

17. The method according to claim 10, wherein the any one of an organ and a body part is completely derived from the target pluripotent stem cell.

18. Use of an animal, which includes a gene coding for a factor which causes a deficiency of any one of an organ and a body part and gives any one of no possibility of survival and difficulty in survival if the factor functions, and in which the any one of an organ and a body part is complemented by complementation, for production of the any one of an organ and a body part.

19. A set for producing any one of a target organ and a target body part, the set comprising:
A) an animal, which includes a gene coding for a factor which causes a deficiency of any one of an organ and a body part and gives any one of no possibility of survival and difficulty in survival if the factor functions, and in which the any one of an organ and a body part is complemented by complementation; and
B) a cell of a same type as the any one of a target organ and a body part.

20. Use of any one of:
a combination of a pluripotent stem cell and nucleic acids coding for a gene coding for a factor which causes a deficiency of any one of an organ and a body part and gives any one of no possibility of survival and difficulty in survival if the factor functions; and
a combination of a pluripotent stem cell which has a gene coding for a factor which causes a deficiency of any one of an organ and a body part and gives any one of no possibility of survival and difficulty in survival if the factor functions and a cell which does not have the gene, the use being for production of the animal according to claim 1.
